# EUROPEAN PATENT APPLICATION

(11) **EP 4 733 324 A2**
(43) Date of publication of application: **29.04.2026**
(21) Application number: 25209159.0
(22) Date of filing: 18.02.2020
(51) Int. Cl.: C07K 16/28

(54) **METHOD OF PRODUCING A BINDER-TOXIN FUSION PROTEIN IN A PLANT CELL OR A WHOLE PLANT**

(30) Priority: 18.02.2019 EP 19157839
(62) Divisional of application: 20704343.1
(71) Applicant: ATB Therapeutics, 6900 Aye (BE)
(72) Inventor: MAGY, Bertrand, 5024 Marche-les-Dames (BE); HOURY, Max, 5081 Saint-Denis (BE)
(74) Representative: Michalski Hüttermann & Partner Patentanwälte mbB

(57) **Abstract**

The present invention relates to a method of producing a binder-toxin fusion protein comprising at least, one protein binder selected from the group consisting of an antibody, an antibody fragment or derivative retaining target binding capacity, or an antibody mimetic, optionally, a peptide linker, and at least one protein toxin or protein protoxin. The method comprises the steps of: contacting a plant cell or a whole plant with a nucleic acid construct comprising in operational linkage at least the following (A) at least one polynucleotide encoding for the protein binder, or a target binding chain or domain thereof, and either B1) a polynucleotide encoding for a cleavable peptide linker and a polynucleotide encoding for a protein toxin, or B2) a polynucleotide encoding for a protein protoxin, which protoxin comprises a cleavable domain for activation thereof, allowing the construct to integrate into the nucleus of the plant cell, or of one or more cells of the whole plant, and expressing the fusion protein encoded by the nucleic acid construct (Fig. 7).

## Description

### Field of the invention

The present application relates to the field of a binder-toxin fusion proteins

### Background

Conjugates combining a target binder and a toxin have been developed forty years ago and now represent a major hope to fight cancer. These conjugates are mainly represented by the class of Antibody-Drug-Conjugates (ADC), consisting of a monoclonal antibody chemically conjugated to a chemical cytotoxic agent via a linker. These drugs combine the specificity of monoclonal antibodies to target cancer cells with the high toxic potency of the payload, to kill targeted cells, while sparing healthy tissues.

However, the concept has proved difficult to translate into clinical success. Despite their specificity to tumor cells, adverse effects frequently occur without that products have reached their effective therapeutic dose, resulting in a relatively narrow therapeutic window which may limit their clinical response. Dose-limiting toxicity was typically observed in non-targeted-expressing tissues (off-target effects), mainly due to the unwanted release of toxin due to the relative instability of the linkers used, rather than due to specificity problems of the antibody (Drake and Rabuka (2015).

In addition to off-target toxicity, it has been shown that only a very few percentage (1-2%) of intact conjugates reaches the targeted tumor (Peters and Brown (2015). Due to this poor targeting efficiency, the cytotoxic payload must have high potency, to still evoke targeted cell death at low doses.

Yet, because of the above described relative instability of the linkers, the use of high potency toxins requires linkers which must be stable within the bloodstream, to avoid premature toxin release, so as to widen the therapeutic window (Parslow et al. (2016)).

Chemically conjugated toxic payloads bear the risk of undesired dissociation from the chemical linker leading to off target toxicity (Alewine et al. (2014)). This instability limits the efficacy of immunoconjugates manufactured that way.

One approach to widen the therapeutic window of antibody drug conjugates is the development of antibodies fused to highly cytotoxic proteins or peptides, mainly from plants and bacteria. This approach was initially developed in the early 1980s. A first generation of such conjugates consisted of cytotoxic peptides chemically coupled to an antibody.

However, the already discussed relative instability of the chemical conjugation, combined with the high immunogenicity of the native cytotoxic proteins, was considered a major obstacle that withstood the therapeutic usability of these conjugates.

However, these conjugates have, in theory, enormous potential for their use in therapy. The mechanism of action of most protein toxins is based on protein synthesis inhibition, which differs from the organic cytotoxins commonly used (mainly, tubulin inhibitors or RNA polymerase inhibitors), meaning non-overlapping toxicity profile, and facilitated combination with standard therapies.

Further, cytotoxic proteins appear to be efficient also in chemo-refractory patients, suggesting that they are not affected by tumor resistance mechanisms observed for organic cytotoxins.

Further, unlike most organic cytotoxins, cytotoxic proteins are also effective against quiescent cells, i.e., cells which are non-dividing.

Further, cytotoxic proteins can be coexpressed with an antibody, namely in the form of a fusion protein. Such recombinant production of a fusion proteins reduces undesirable payload dissociation observed for chemical linker technologies.

Genetically fusing a protein binder, e.g., an antibody, to a cytotoxic protein via a peptide linker provides a couple of advantages. Besides the mere linking role, linkers may affect folding, stability, pharmacokinetic profile and biological activity of the fusion protein, as well as its production yield in the host cells.

Two categories of linker exist, namely stable and cleavable linkers. Stable linkers consist of stable peptide sequence which may have a prolonged plasma half-life and avoid unintended release of the cytotoxic protein. The entire conjugate is internalized into the cell, and then the toxin is released by intracellular degradation of the protein binder.

Many mammalian protease sensitive sequences can be used as linkers in the design of cleavable bio-conjugates. In particular, sequences sensitive to enzymes overexpressed by cancer cells can be used to activate cytotoxic fusion protein into targeted cells or in the tumoral environment. Yet, these complex fusion proteins using a mammalian enzyme sensitive sequence are difficult to produce into standard systems (mammalian cells (e.g. CHO or HEK), insect cells and yeasts) mainly because of the presence of the specific enzyme even with a low background expression. In this case, the propeptide is activated by the release of its active domain, inducing inhibition of host cell proliferation. Consequently, binder-toxin fusion proteins with mammalian proteolytic cleavable linkers must be produced in bacteria expression systems. However, bacteria are unable to properly fold complex proteins with multiple domains and lack the ability to form disulphide bonds (Yin et al. (2007)). These limitations restrain the bacteria system to the production of single chain antibody fragment (scFv) and aglycosylated cytotoxic protein based binder-toxin fusion proteins. However, the small size of bacterial scFv based bio-conjugates induces rapid renal clearance, limiting the therapeutic window of these molecules (Guo et al. (2016).

WO2009064815 discloses that algae chloroplast contains the necessary machinery to fold complex binder-toxin fusion proteins avoiding host cell killing as observed into standard system. Indeed, the alga *Chlamydomonas reinhardtii* have a single chloroplast like those found in prokaryotes but containing proteins allowing the folding of complex proteins (protein disulphide isomerase, chaperones). WO2009064815 demonstrated the suitability of the green alga chloroplast to produce soluble and functional single chain antibody (CD22) with hinge, CH2, CH3 domains of a human IgG1 fused to a protein toxin by a non-cleavable linker consisting of two repeated G4S sequence (four glycine's followed by a serine). However, one major concern is the post-translational modification of micro-algae chloroplasts, particularly the lack of enzymatic machinery for N-glycosylation, a critical attribute for biopharmaceuticals (Mathieu-Rivet et al. (2014)).

Another approach based on human embryonic kidney cells (HEK-293T) was developed using an uncleavable binder-toxin fusion protein composed of a Vascular endothelial growth factor 121 (VEG121) linked via G4S stable linker to a protected Granzyme B (Mohamedali et al. (2013)). The functional domain of protease Granzyme B is protected by an extra histidine tag linked by an enterokinase sensitive site most probably to avoid host cell killing. After production, the protected site must be removed by an additional step of enterokinase treatment. Even though an anti-tumor efficacy was observed, the production of this kind of binder-toxin fusion protein is complex.

Said limitation can be overcome by using a fully recombinant bioconjugation of a targeting moiety linked via a peptide linker to a peptide payload. Such recombinant immunoconjugate has for example been designed using a CD20-specific single chain variable fragment (scFv) conjugated to a modified shiga-like toxin, and expressed in a prokaryotic expression system (WO2014164680A1). The resulting compound displayed promising results in Phase I/Ib in NHL, demonstrating that a full recombinant immunoconjugate can reduce the off-target toxicity observed for antibodies chemically conjugated to a peptide toxin.

However, scFv antibodies based immunoconjugates have been shown to have a very limited blood half-life due to renal clearance which limits their efficacy, while bacterial models often used to produce scFv based immunotoxins are not suitable to produce full length antibodies or scFv-Fc structures due to their inability to allow the formation of disulfide bridges. Further, such bacterial systems do not glycosylate the antibodies or antibody fragments produced therewith, which may likewise lead to reduced half-life or reduces effector functions of the antibodies produced. For these reasons, typically, mammalian expression systems like CHO (Chinese hamster ovary cells) are being used to produce such complex antibodies or antibody fragments or derivatives.

However, production of an active protein toxin payload, or a conjugate comprising the latter, is hardly achievable in mammalian cells, due to toxicity reasons, in particular if a cleavage site is used that is recognized by a mammalian protease. However, it is of great interest to have, in an immunoconjugate, such cleavage site that is recognized by a mammalian protease.

This would allow to activate the toxin after the binder-toxin fusion protein has bound to its target, e.g., at the site of disease characterized by said target. Due to its susceptibility to mammalian proteases, the cleavage site will be cleaved and the thus activated toxin will be released.

It is hence one object of the present invention to provide an efficient production system to harness the therapeutic potential of new binder-toxin fusion proteins.

It is one further object of the present invention to provide a method for the production of a binder-toxin fusion protein that
a) allows the formation of disulfide bridges in the binder protein and/or the glycosylation of the binder protein
b) allows the use of a protein toxin that is toxic to mammalian cells, and/or
c) allows the incorporation of a cleavage site that is recognized by a mammalian protease.

It is yet another object of the present invention to enable the production of glycosylated binder-toxin fusion proteins combining a targeting moiety recombinantly fused to a cytotoxic protein which is activated after linker cleavage, with a prolonged serum half-life.

Any of these objects would be desirable to be solved even if the actual binder protein (a) is not glycosylated, (b) uses a toxin that is not toxic to mammalian cells, or (c) does not have such cleavage site, because the mere fact that such method allows any of the three provides high flexibility, and allows a large array of binder-toxin fusion proteins to be produced.

These and further objects are met with methods and means according to the independent claims of the present invention. The dependent claims are related to specific embodiments.

### Summary of the Invention

The present invention provides a method to produce a binder-toxin fusion protein. The invention and general advantages of its features will be discussed in detail below.

### Brief Description of the Figures

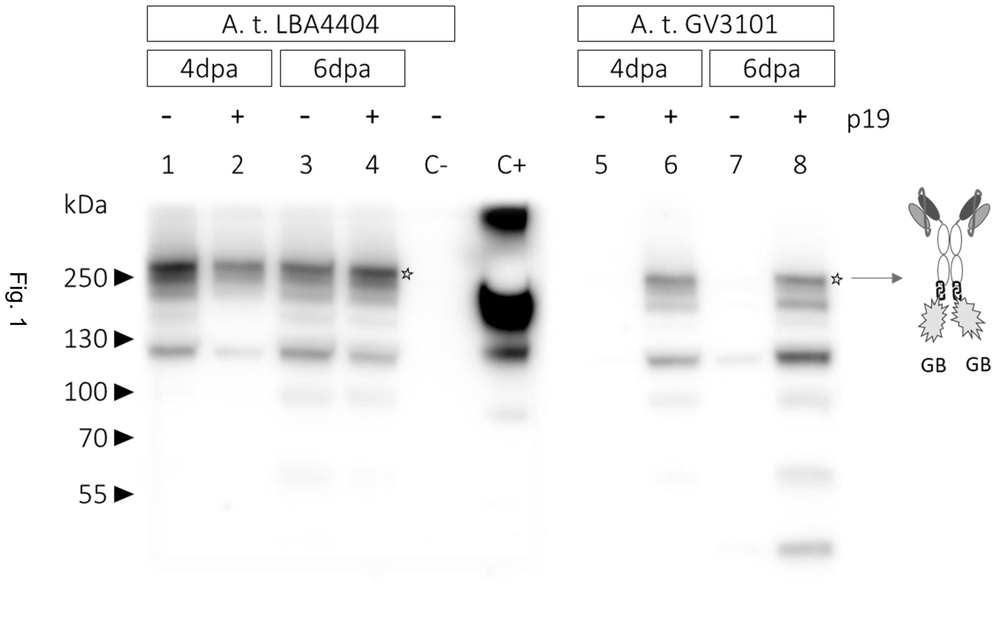

Figure 1. 30 µg of *Nicotiana benthamiana* leaf extracts after 4 and 6 days post agroinfiltration (4 or 6 dpa) were analyzed by westernblotting using anti human IgG Fc part antibodies. The binary transformation vector containing the p19 silencing suppressor gene (+) or not (-) were used. *Agrobacterium tumefaciens* (A. t.) LBA4404 (lane 1 to 4) or GV3101 (lane 5 to 8) were used to express the fusion protein, strains are indicated on the top of the figure. A negative control using an empty pPZP-ATB binary plasmid into A. t. LBA4404 (C-). 200 ng of human serum IgGs (Sigma, I5154) were used as a positive control (C+). The 4-20% acrylamide SDS-PAGE were performed under non-reducing conditions. Integral protein size is indicated by a star.

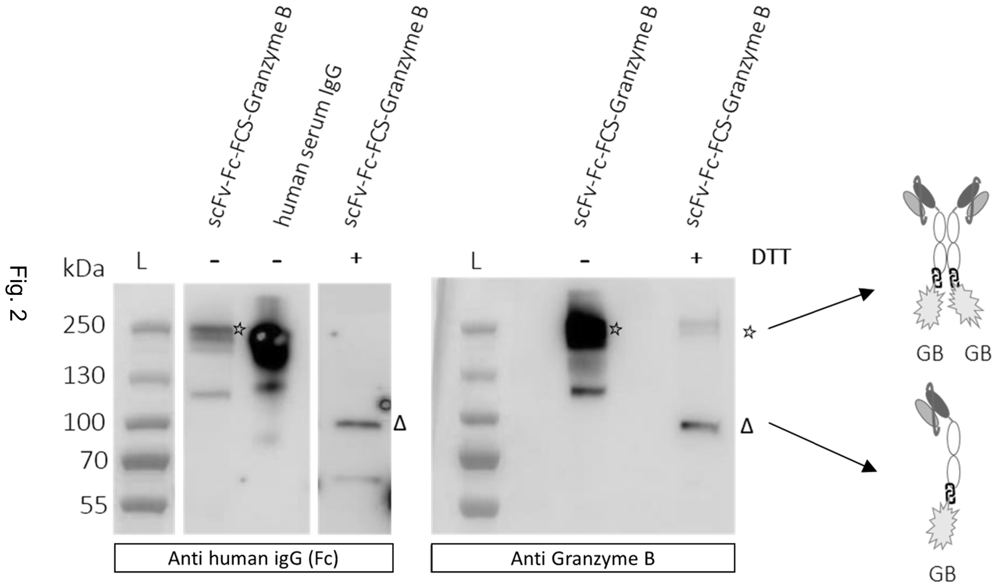

Figure 2. 25 µg of *Nicotiana benthamiana* leaf extracts after 4 days post agroinfiltration were analyzed by western blotting using anti human IgG Fc part and anti-human (left panel) Granzyme B (right panel) antibodies. *Agrobacterium tumefaciens* (A. t.) LBA4404 without p19 silencing suppressor gene were used to express the fusion protein. 50 ng of human serum IgGs (Sigma, I5154) were used as a positive control (C+). The 4-20% acrylamide SDS-PAGE was performed under reducing (+ DTT) or non-reducing conditions (- DTT). L indicate the protein ladder (molecular-weight size marker). Integral protein size is indicated by a star. Monomeric integral size is indicated by Δ.

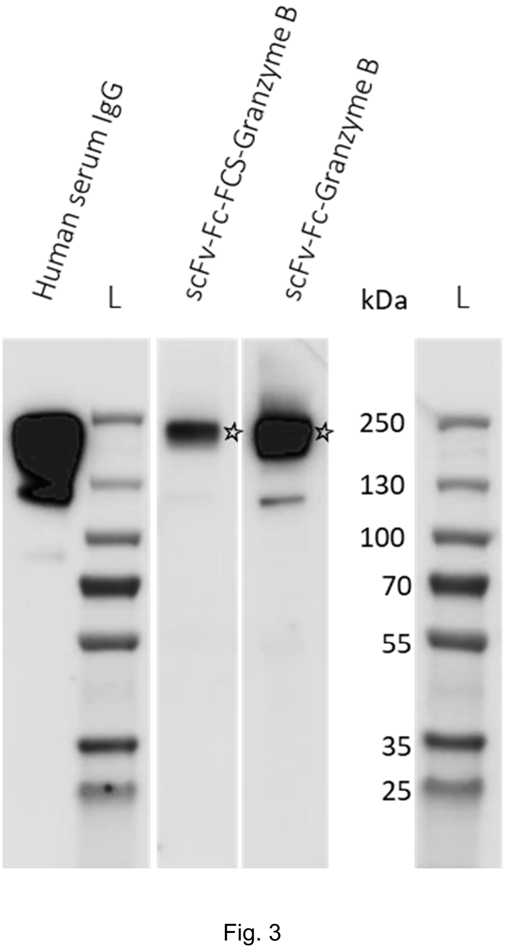

Figure 3. 40 µg of *Nicotiana benthamiana* leaf extracts after 4 days post agroinfiltration were analyzed by western blotting using anti human IgG Fc part antibodies. 50 ng of human serum IgGs (Sigma, 15154) were used as a positive control (Human serum IgG). The 4-20% acrylamide SDS-PAGE was performed under non-reducing conditions. Expressed constructs are indicated on top of the corresponding lane. L indicates the protein ladder (molecular-weight size marker). Integral protein size is indicated by a star.

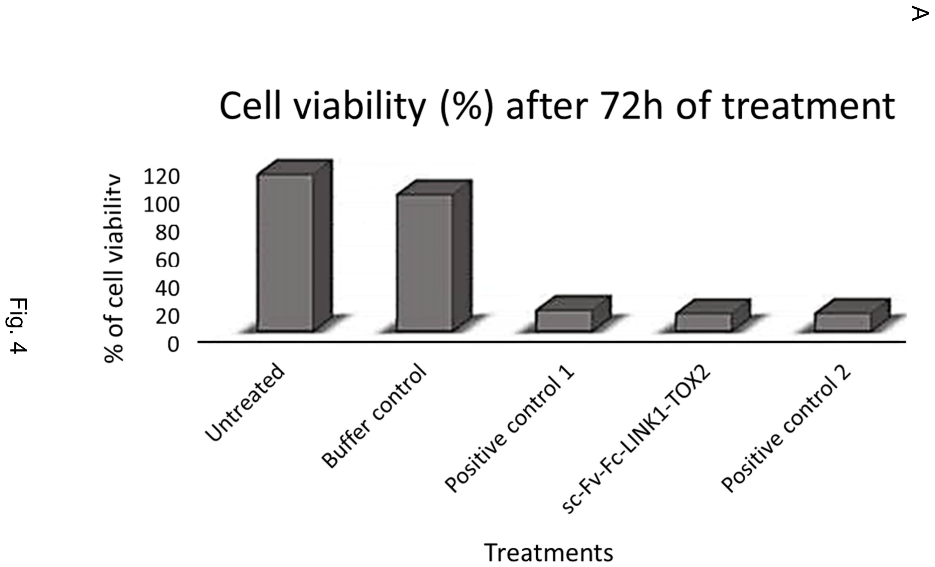

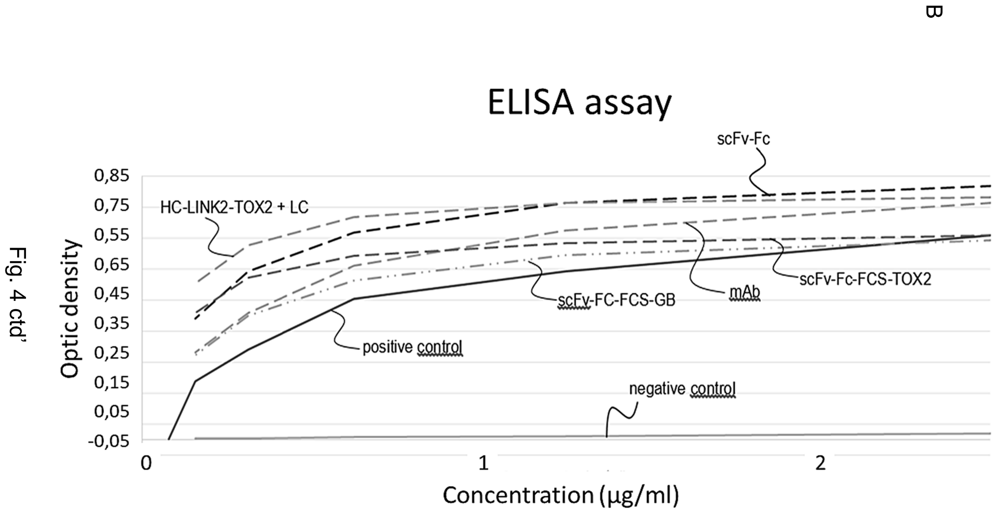

Figure 4. *In vitro* cytotoxicity and binding
A: In vitro cytotoxicity by WST-1 assay. 10 µl of purified scFv-Fc-LINK1-TOX2, wherein LINK1 is cleavable by the subtilisin-like proprotein convertase family, the TOX2 inhibit protein synthesis and the scFv-Fc bind a cell surface protein expressed on B cells, have been diluted into 40 µl of growth medium before to be added on Raji cells. Viability have been observed by optical density reading 72 hours after incubation. DMSO (5%) or triton (2%), serve as positive controls. Buffer control only have been used (10µl into 40 µl of growth medium) to standardize the effect of scFv-Fc-LINK1-TOX2 and positive controls.

| | Untreated | Buffer control | Positive control 1 | scFv-Fc-LINK1-TOX2 | Positive control 2 |
|---|---|---|---|---|---|
| % viability (standardisation 100% PBS) | 114,814815 | 100 | 15,20467836 | 12,80701754 | 12,98245614 |

B: Binding capacity analysis by ELISA. Antigen have been coated on 96 well microplate. 50 µl of purified constructions have been incubated for 1 hour. Goat anti- human Fc antibody HRPO have been added for the detection. 50 µl of specific naked mAb have been used as positive control (linear red curve). 50µl of nonspecific naked mAb have been used as negative control (linear grey curve). FCS = Furin cleavage site, GB = Granzyme B

| | Positive control | Negative control | mAb | scFv-Fc | scFv-Fc-FCS-GB | HC-LINK2-TOX2 + LC | scFv-Fc-FCS-TOX2 |
|---|---|---|---|---|---|---|---|
| 2,5 | 0,73715 | 0,0198 | 0,7619 | 0,8166 | 0,6431 | 0,7819 | 0,6595 |
| 1,25 | 0,65905 | 0,0112 | 0,6754 | 0,7639 | 0,5946 | 0,7625 | 0,6329 |
| 0,625 | 0,54315 | 0,0081 | 0,5611 | 0,6674 | 0,513 | 0,7179 | 0,5933 |
| 0,313 | 0,4548 | 0,0053 | 0,4084 | 0,5429 | 0,4001 | 0,6266 | 0,5226 |
| 0,156 | 0,2911 | 0,0045 | 0,2812 | 0,3896 | 0,2725 | 0,5039 | 0,409 |
| 0,0781 | 0,18805 | | | | | | |
| 0 | 0,0017 | | | | | | |

Figure 4. 25 µg of *Nicotiana tabacum* plant cells extracts after 3 days post cocultivation were analyzed by westernblotting using anti human IgG Fc part and anti-human (left panel) Granzyme B (right panel) antibodies. *Agrobacterium tumefaciens* (A. t.) LBA4404 harboring binary plasmids with p19 silencing suppressor gene were used to express the fusion protein. The 4-20% acrylamide SDS-PAGE was performed under non-reducing conditions. L indicate the protein ladder (molecular-weight size marker). Integral protein size is indicated by a star.

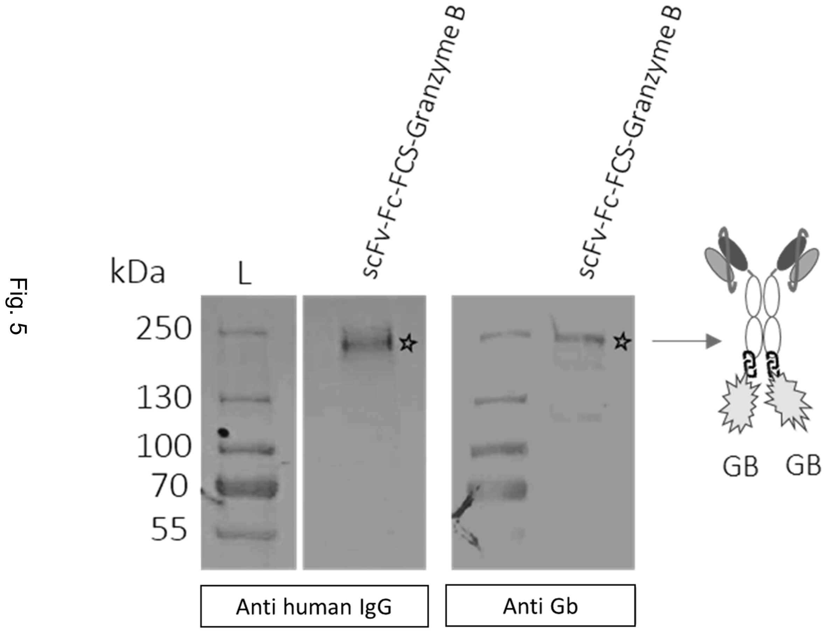

Figure 5. 33 µL of *Nicotiana tabacum* 5 days old stable plant cells extracts were analyzed by westernblotting using anti human IgG Fc part antibody. A selected plant cell line stably expressing scFv-Fc-FCS-Granzyme B has been used for cell extract analysis. The 4-20% acrylamide SDS-PAGE was performed under non-reducing conditions. L indicate the protein ladder (molecular-weight size marker). Integral protein size is indicated by a star.

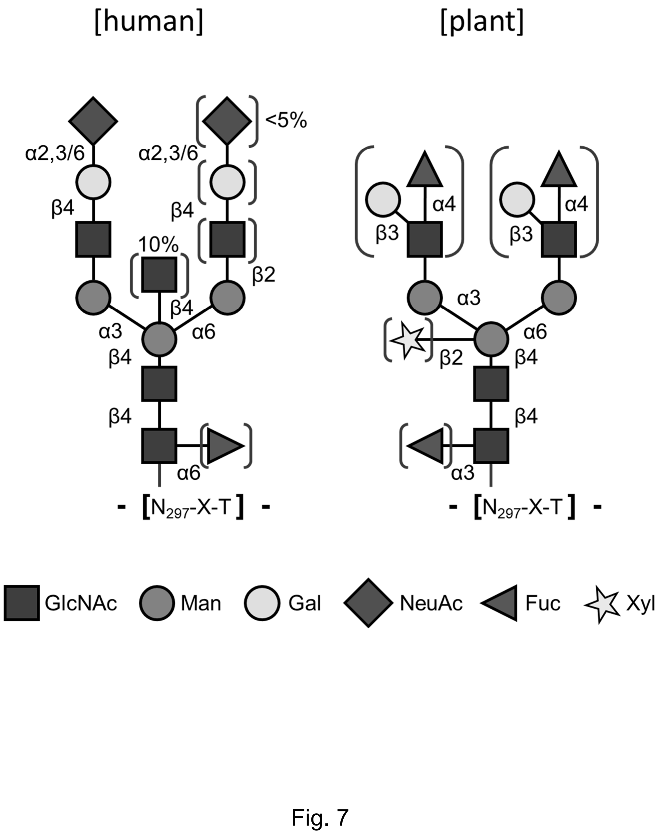

Figure 7. N-glycosylation patterns produced by human cells (left) and tobacco plants or cells (right).

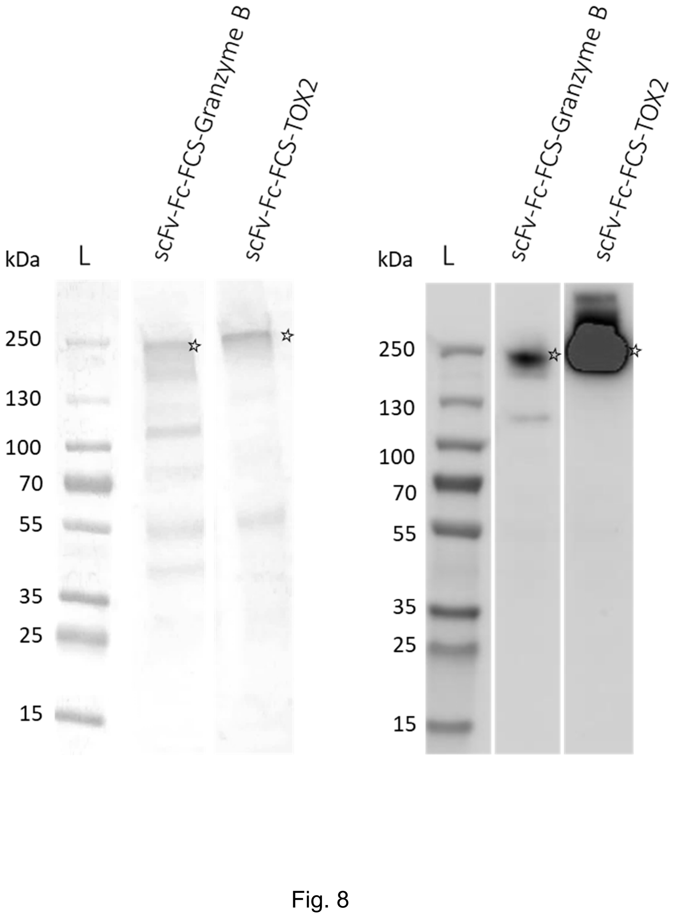

Figure 8. (Left panel) 33 µL of *Nicotiana tabacum* plant cells extracts after 3 days post cocultivation were analyzed by westernblotting using anti human IgG Fc antibody. *Agrobacterium tumefaciens* (A. t.) LBA4404 harboring binary plasmids with p19 silencing suppressor gene were used to express the fusion protein. (Right panel) 40 µg of *Nicotiana benthamiana* leaf extracts after 4 days post agroinfiltration were analyzed by western blotting using anti human IgG Fc part antibodies. The 4-20% acrylamide SDS-PAGE were performed under non-reducing conditions. Expressed constructs are indicated on top of the corresponding lane. TOX2 indicates a toxin from toxin class 2 as disclosed herein (protein synthesis inhibitor). L indicates the protein ladder (molecular-weight size marker). Integral protein size is indicated by a star.

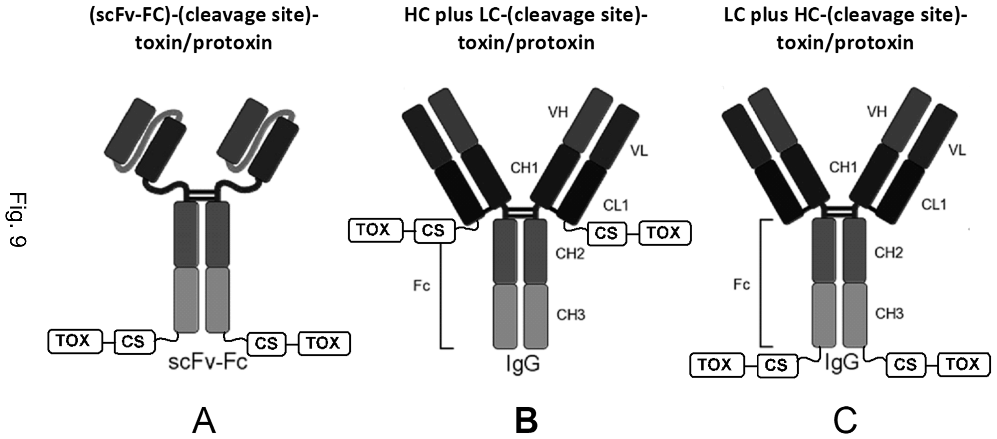

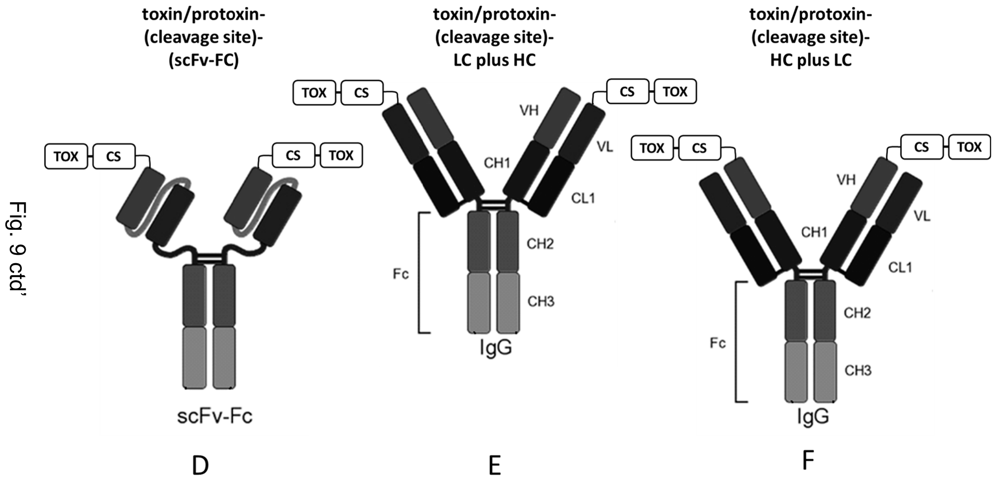

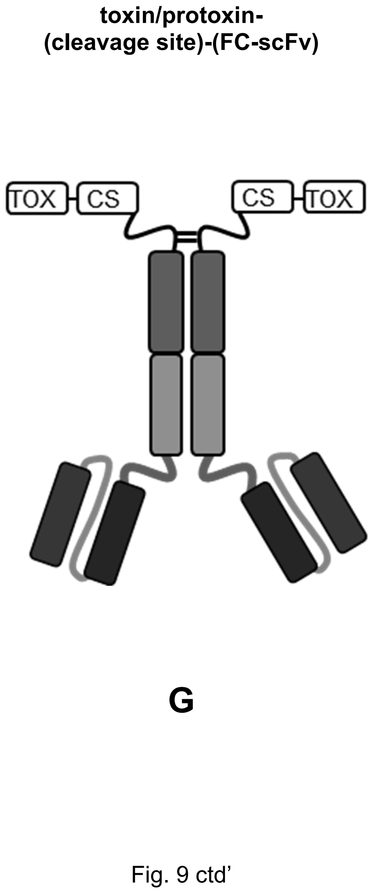

Figure 9: Structures of binder-toxin formats according to the invention.
9A-C: Toxin is fused to a C-terminus of an antibody chain. A: (scFv-FC)-(cleavage site)-toxin/protoxin; B: HC plus LC-(cleavage site)-toxin/protoxin, and C: LC plus HC-(cleavage site)-toxin/protoxin.
9D-G Toxin is fused to an N-terminus of an antibody chain.
scFv-FC is a specific antibody format as discussed herein. LC is the light chain of an IgG antibody. HC is the heavy chain of an IgG antibody. CS means cleavage site, and Tox means toxin/protoxin. The bars between the different chains symbolize disulfide bonds.

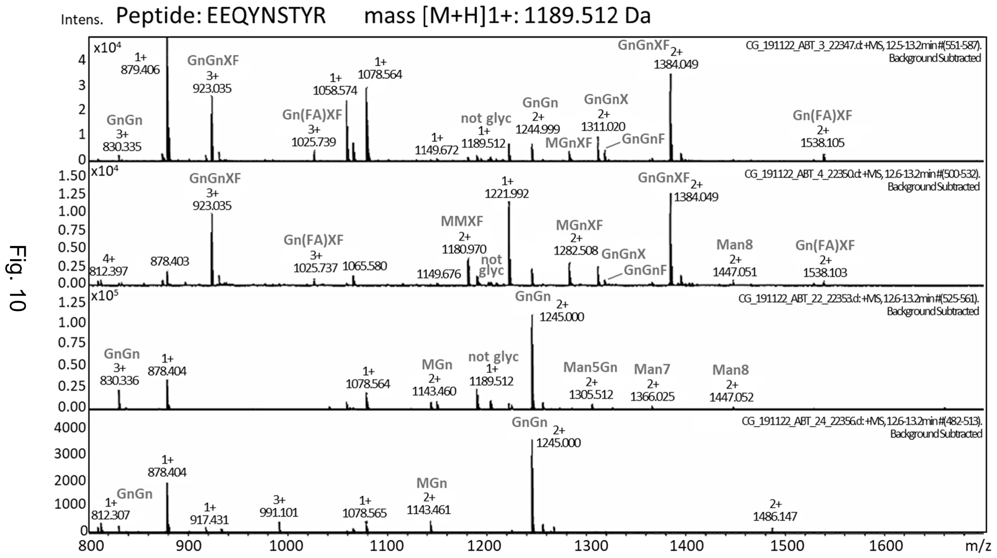

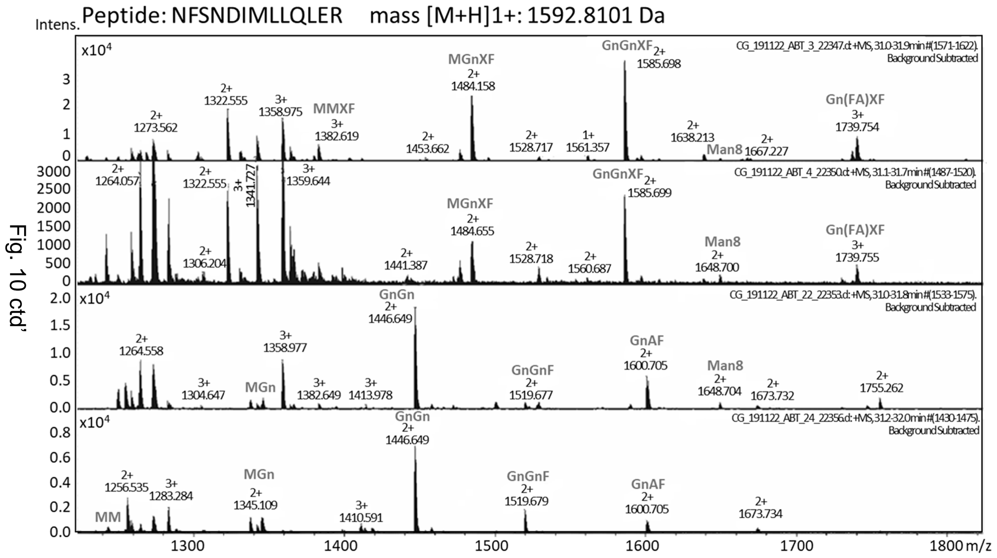

Figure 10: Results of peptide glycoform analysis. To demonstrate that peptides taken from the antibodies disclosed herein and comprising an N-glycosylation site have characteristic glycoforms which separate them from proteins produced in mammals, different strains of *Nicotiana benthamiana* were used, some of which being glycoengineered by RNA interference (RNAi) technology (Material provided by NOMAD Bioscience GmbH, Munich) , to obtain a targeted down-regulation of the endogenous β-1,2-xylosyltransferase (XylT) and α 1,3-fucosyltransferase (FucT), Fig, 10 shows the MS spectra of two such peptides as produced in the different strains.

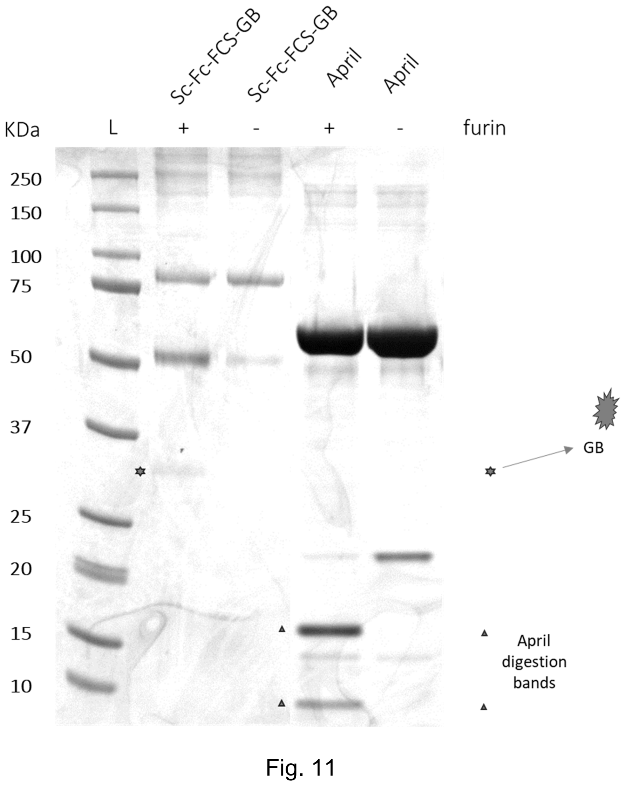

Figure 11: Results of cleavage assay. scFv-Fc-FCS-GB (FCS = Furin cleavage site, GB = Granzyme B) have been expressed from *Nicotiana benthamiana.* Protein have been purified by protein A chromatography from leaf extracts after 4 days post agroinfiltration. Purified material have been exposed to recombinant furin. The 4-20% acrylamide SDS-PAGE was performed under reducing conditions. L indicate the protein ladder (molecular-weight size marker). Resulting free GB protein after cleavage is indicated by a star. Recombinant protein April was used as cleavage control. Cleavage related band of April are indicated by a Δ.

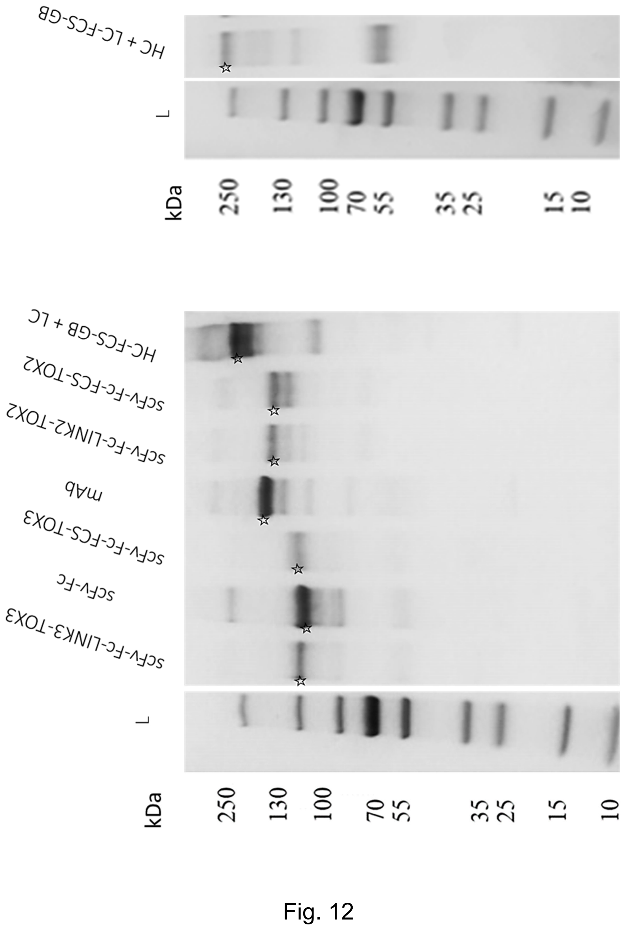

Figure 12: Purified protein analysis by SDS PAGE Coomassie blue. Several constructions have been expressed from *Nicotiana benthamiana.* Protein have been purified by protein A chromatography from leaf extracts after 4 days post agroinfiltration.7 µl of purified protein have been added to 7 µl of loading buffer (2x). Then 12 µl have been loaded into respective well. The 4-20% acrylamide SDS-PAGE was performed under non-reducing conditions. L indicates the protein ladder (molecular-weight size marker). Integral protein size is indicated by a star.

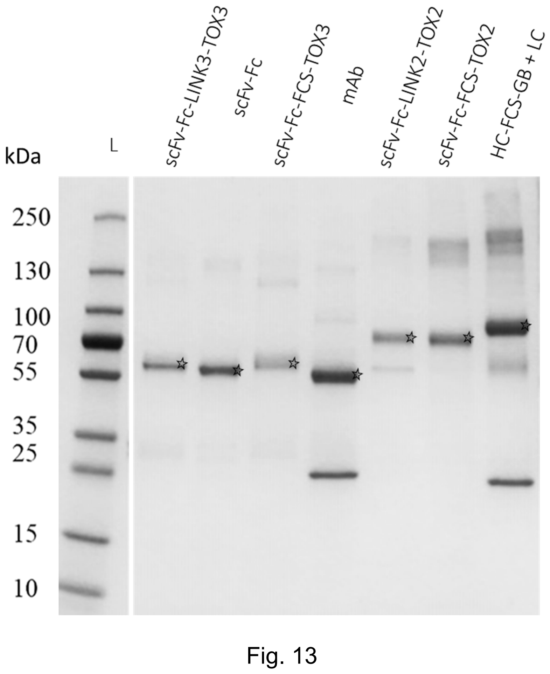

Figure 13: Purified protein analysis by SDS PAGE Coomassie blue. Several constructions have been expressed from *Nicotiana benthamiana.* Protein have been purified by protein A chromatography from leaf extracts after 4 days post agroinfiltration. 7 µl of purified protein have been added to 7 µl of loading buffer (2x). Then 12 µl have been loaded into respective well. The 4-20% acrylamide SDS-PAGE was performed under reducing conditions. L indicates the protein ladder (molecular-weight size marker). Integral protein size is indicated by a star.

### Detailed Description of the Invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a", "an", and "the" include singular and/or plural referents unless the context clearly dictates otherwise. It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure, and avoid lengthy repetitions.

According to a first aspect of the invention, a method of producing a binder-toxin fusion protein is provided,
a) one protein binder selected from the group consisting of
   - an antibody
   - an antibody fragment or derivative retaining target binding capacity, or
   - an antibody mimetic,
b) optionally, a peptide linker, and
c) at least one protein toxin or protein protoxin
said method comprising the steps of:
(i) contacting a plant cell or a whole plant with a nucleic acid construct comprising in operational linkage at least the following
   A) at least one polynucleotide encoding for the protein binder, or a target binding chain or domain thereof, and either
      B1) a polynucleotide encoding for a cleavable peptide linker and a polynucleotide encoding for a protein toxin, or
      B2) a polynucleotide encoding for a protein protoxin, which protoxin comprises a cleavable domain for activation thereof,
(ii) allowing the construct to integrate into the nucleus of the plant cell, or of one or more cells of the whole plant, and
(iii) expressing the fusion protein encoded by the nucleic acid construct.

The inventors found, surprisingly, that with such method, recombinant binder-toxin fusion proteins can be produced at large scale and with high productivity.

As used herein, the term "plant" (including the cells derived therefrom) relates to algae (including Chlorophyta and Charophyta/Streptophyta, as well as Mesostigmatophyceae, Chlorokybophyceae and Spirotaenia), and also to land plants (Embryophytes), including Gymnospertms and Angiosperms, including Mono- and Dicotyledonae.

In one embodiment, the plant or plant cell with which the nucleic acid construct is contacted is not a chloroplast, or not a chloroplast of an algae, in particular not the chloroplast of *Chlamydomonas reinhardtii.* In another embodiment, structure in the plant or plant cell with which the nucleic acid construct is contacted is not a chloroplast, or not a chloroplast of an algae, in particular not the chloroplast of *Chlamydomonas reinhardtii.*

As used herein, the term "protein toxin" or "protein protoxin" is meant to encompass cytotoxic and/or cytostatic proteins. or the pro-variants thereof.

As used herein the term "cytostatic protein" refers to a protein that can inhibit cell proliferation or cell division without necessarily killing the cell. Suitably, the cytostatic agent inhibits the proliferation of tumor cells.

As used herein, the term "cytotoxic protein" refers to a protein that is harmful to cells and ultimately causes cell death. In some embodiments, the cytotoxic protein harms rapidly dividing cells such as tumor cells and causes tumor cell death, especially tumor cell death while not causing damage to or causing less damage to non-tumor cells.

The terms "protein toxin" or "protein protoxin", refer without limitation to toxins that are, by their chemical nature, proteins (i.e., peptides having a length of ≥ 50 amino acid residues) or polypeptides (i.e., peptides having a length of ≥ 10 - ≤ 50 amino acid residues). A protoxin, in the meaning of the present invention, is a precursor of a toxin, also called a latent toxin, which needs to be activated, e.g., by cleaving off an inhibitory amino acid sequence, or by undergoing a conformational change. The terms "protoxin" and "protein protoxin" are used interchangeably here and mean the same subject matter.

Plant-based recombinant protein expression has been developed for 3 decades. Today, several therapeutic proteins, such as monoclonal antibodies (mAbs), produced in plants or plant cells (such as tobacco or tobacco cells) have been tested in clinical trials and are commercialized or are close to be (Yao et al. (2015)). Plant-based recombinant protein expression, including antibodies. can also be carried out in algae. (Hempel et al., 2011)

Plants have a couple of advantages over prokaryotic and eukaryotic cells systems regarding their low cost of production, inherent product safety, easy upscaling, their ability to fold and assemble complex proteins and to carry out complex post-translational modifications.

Besides, plant suspension cells cultures offer increased reproducibility and safety during production (no known microbes, insects or mammalian pathogens), and meets current good manufacturing practice production requirements. Plant suspension cells cultures only require simple defined nutrients to grow, offering much less expensive operational costs than mammalian or microbial systems.

The term "fusion protein" as used herein refers to a protein that has a peptide component operably linked to at least one additional component and that differs from a natural protein in the composition and/or organization of its domains.

The term "operably linked" as used herein, when referring to two or more polynucleotides, means a situation when the different polynucleotides are placed in a functional relationship with one another. For instance, a promoter is operably linked to a coding sequence if the promoter effects the transcription of the coding sequence. Likewise, the coding sequence of a signal peptide is operably linked to the coding sequence of a polypeptide if the signal peptide effects the extracellular secretion of that polypeptide. According to one embodiment of the present invention, when the respective polynucleotides encode different peptides, "operably linked" means that the respective polynucleotides are contiguous and, where necessary to join two protein coding regions, the open reading frames are aligned.

The term "cleavable peptide linker" as used herein refers to an internal amino acid sequence within the fusion protein which contains residues linking the binder moiety and toxin protein so as to render the toxin protein incapable of exerting its toxic effect outside the target cell or limiting its ability of toxin protein to inhibit cell growth (cytostasis) or to cause cell death (cytotoxicity). In such way, the protein toxin is maintained inactive as long as it is in the plasma, until it reaches the target cell, where the cytotoxic payload will be selectively released and/or activated (Grawunder & Stein, 2017). Inside the target cell, the cleavable linker sequence is cleaved and the toxin protein becomes active or toxic. The fusion protein of the invention is composed of a cell-specific binder moiety and an protein toxin moiety linked by a a specific amino acid residue or amino acid sequence that has cleavage recognition site for specific proteases, particularly but not limited to cancer specific protease, and/or are cleavable under specifics conditions such as, without limitation, acid and/or reducing conditions. Sequences encoding cleavage recognition sites for specific protease may be identified among known ubiquitous human protease and/or by testing the expression of cancer associate protease. Also the linker sequence should not interfere with the role of the binder moiety in cell binding and internalization into lysosomes.

The term "cleavable domain" of a protoxin relates to a sequence that, once cleaved by hydrolysis or enzymatic cleavage, activates the toxin part of the protoxin. Many protoxins have an amino acid domain that is specifically cleaved by an enzyme, or by pH dependent hydrolysis (e.g. after endocytosis in the endosomes), so as to release the active toxin part into the cytosol. Such cleavable domains double act as "naturally occurring" cleavable peptide linkers (or "intrinsic cleavage sites"), contrary to the cleavable peptide linkers which have to be used in case the toxin does not comprise a cleavable domain for activation, e.g., because it does not come as a protoxin.

The term "selective activation/release" refers to the activation of ability of protoxin or protein toxin to inhibit cell growth (cytostasis) or to cause cell death (cytotoxicity) under particular conditions. This selective activation refers to an unnatural or not naturally found modifiable activation moiety of a protein toxin that, upon modification, converts inactive toxin (protein protoxin) into an active toxin or a native cleavable linker of a protoxin. When the selectively modifiable activation moiety is a component of the toxin fusion protein, modification of the modifiable activation moiety can result directly in the protoxin becoming toxic to the target cell, or can result in the protoxin assuming a form that is natively activatable to become toxic to the target cell. Natively activatable protoxins comprise, for example, modification of the modifiable activation moiety such that it is sensitive to endogenous components of the target cell, or the environment surrounding the target cells. (e.g., a target cell specific protease or a ubiquitous protease), and/or specific conditions such as, without limitation, acid and/or reducing conditions. Natively active toxin can be modified to be inactive (protoxin) into the toxin fusion using natural or unnatural and not naturally found modifiable activation moiety such that it is sensitive to endogenous components of the target cell, or the environment surrounding the target cells and/or specific conditions such as, without limitation, acid and/or reducing conditions. Modifiable activation moiety is defined as cleavable linker in the present invention.

Hence, while a cleavable linker provides clear advantages over a stable linker as regards the activity profile, the use thereof complicates the production of respective binding protein-toxin conjugates in mammalian, insect and yeast cells, because cleavage of the linker leads to self-intoxication of the production system. This, however, does not apply to plant-based production systems, because
(i) they don't cleave the linker (due to lack of respective proteases or reducing/hydrolyzing conditions) and/or
(ii) the respective protein toxin which is toxic to mammals or mammalian cells is not toxic to plants or plant cells.

As used herein, the term "monoclonal antibody", shall refer to an antibody composition having a homogenous antibody population, i.e., a homogeneous population consisting of a whole immunoglobulin, or an antigen binding fragment or derivative thereof. Particularly preferred, such antibody is selected from the group consisting of IgG, IgD, IgE, IgA and/or IgM, or a fragment or derivative thereof.

As used herein, the term "fragment" shall refer to fragments of such antibody retaining target binding capacities, e.g.
- a CDR (complementarity determining region),
- a hypervariable region,
- a variable domain (Fv),
- an IgG heavy chain (consisting of VH, CH1, hinge, CH2 and CH3 regions),
- an IgG light chain (consisting of VL and CL regions), and/or
- a Fab and/or F(ab)₂.

As used herein, the term "derivative" shall refer to protein constructs being structurally different from, but still having some structural relationship to the common antibody concept, e.g., scFv, scFv-FC, Fab and/or F(ab)₂, as well as bi-, tri- or higher specific antibody constructs or monovalent antibodies, and further retaining target binding capacities. All these items are explained below.

Other antibody derivatives known to the skilled person are Diabodies, Camelid Antibodies, Nanobodies, Domain Antibodies, bivalent homodimers with two chains consisting of scFvs, IgAs (two IgG structures joined by a J chain and a secretory component), shark antibodies, antibodies consisting of new world primate framework plus non-new world primate CDR, dimerised constructs comprising CH3+VL+VH, and antibody conjugates (e.g. antibody or fragments or derivatives linked to a toxin, a cytokine, a radioisotope or a label). These types are well described in literature and can be used by the skilled person on the basis of the present disclosure, with adding further inventive activity.

Methods for the production of a hybridoma cell have been previously described (see Köhler and Milstein 1975, incorporated herein by reference). Essentially, e.g., a mouse is immunized with a human soluble Guanylyl Cyclase (sGC) protein, followed by B-cell isolation from said mouse and fusion of the isolated B-cell with a myeloma cell.

Methods for the production and/or selection of chimeric or humanized mAbs are known in the art. Essentially, e.g., the protein sequences from the murine anti sGC antibody which are not involved in target binding are replaced by corresponding human sequences. For example, US6331415 by Genentech describes the production of chimeric antibodies, while US6548640 by Medical Research Council describes CDR grafting techniques and US5859205 by Celltech describes the production of humanised antibodies. All of these disclosures are incorporated herein by reference.

Methods for the production and/or selection of fully human mAbs are known in the art. These can involve the use of a transgenic animal which is immunized with human sGC, or the use of a suitable display technique, like yeast display, phage display, B-cell display or ribosome display, where antibodies from a library are screened against human sGC in a stationary phase.

In vitro antibody libraries are, among others, disclosed in US6300064 by MorphoSys and US6248516 by MRC/Scripps/Stratagene. Phage Display techniques are for example disclosed in US5223409 by Dyax. Transgenic mammal platforms are for example described in EP1480515A2 by TaconicArtemis. All of these disclosures are incorporated herein by reference.

IgG, scFv, scFv-FC, Fab and/or F(ab)₂ are antibody formats well known to the skilled person. Related enabling techniques are available from the respective textbooks.

As used herein, the term "Fab" relates to an IgG fragment comprising the antigen binding region, said fragment being composed of one constant and one variable domain from each heavy and light chain of the antibody.

As used herein, the term "F(ab)₂" relates to an IgG fragment consisting of two Fab fragments connected to one another by one or more disulfide bonds.

As used herein, the term "scFv" relates to a single-chain variable fragment being a fusion of the variable regions of the heavy and light chains of immunoglobulins, linked together with a short linker, usually serine (S) or glycine (G). This chimeric molecule retains the specificity of the original immunoglobulin, despite removal of the constant regions and the introduction of a linker peptide.

As used herein, the term "scFv-FC" relates to a specific antibody format. This format is particularly stable and can be expressed with high yield in plant cells and plants. scFv-FC constructs are for example disclosed in Bujak et al (2014), the content of which is incorporated herein by reference. scFv-Fc constructs are dimeric constructs comprising two chains associated to one another for example by one or more disulfide bonds, wherein each of which consist of a structure as follows (in N->C direction):
V_{L}-linker-V_{H}-Linker-FC, or
V_{H}-linker-V_{L}-Linker-FC
with V_{L} being the variable domain of the light chain of an antibody, V_{H} being the variable domain of the heavy chain of an antibody, and FC being the constant domain of an antibody.

The use of a full-length IgG-shaped antibody or a scFv-Fc binding domain confers a longer half-life to the conjugate. Moreover, the Fc part of the antibody might be of utmost importance when CDC (Complement dependent cytotoxicity) or ADCC (Antibody dependent cellular cytotoxicity) activation is required.

Modified antibody formats are for example bi- or trispecific antibody constructs, antibody-based fusion proteins, immunoconjugates and the like. These types are well described in literature and can be used by the skilled person on the basis of the present disclosure, with adding further inventive activity. Furthermore, also monovalent antibodies have been previously described in US 2004/0033561 A1 (referred to therein as monobodies) or WO2007048037; both of which are incorporated herein by reference.

Antibody mimetics are organic compounds - in most cases recombinant proteins or peptides - that, like antibodies, can specifically bind antigens, but that are not structurally related to antibodies. Common advantages over antibodies are better solubility, tissue penetration, stability towards heat and enzymes, and comparatively low production costs. Antibody mimetics are being developed as therapeutic and diagnostic agents, and encompass, *inter alia,* Affibody molecules, Affilins, Ubiquitins, Affimers, Affitins, Alphabodies, Anticalins, Avimers, DARPins, Fynomers, Kunitz domain peptides, Monobodies and nanoCLAMPs. Antibody mimetics are discussed in great detail, inter alia, in Gebauer and Skerra (2009), incorporated herein by reference.

Generally, the protein binder may consist of a single chain. This is the case, e.g., where the protein binder is a scFv antibody, or a scFv-FC. In this case, the entire protein binder may be encoded on a single polynucleotide.

In another embodiment the protein binder may comprise two or more chains, like e.g. in a full size IgG or in a F(ab)2 fragment. In such case it may be provided that the nucleic acid construct may comprise two or more polynucleotides encoding for the different chains or domains for the protein binder.

In another embodiment where the protein binder comprises two or more chains it may be provided that two nucleic acid constructs are provided, the first comprising the three polynucleotides encoding for the first chain of the protein binder, the linker and the toxin, while the second comprises the polynucleotide encoding for the second chain of the protein binder.

According to one embodiment of the invention, the method further comprises the step of (iv) recovering and/or purifying the fusion protein expressed in step (iii)

According to one other embodiment of the invention, the plant or plant cell is from the genus *Nicotiana.*

In one embodiment, when using a plant, the expression of the fusion protein is a transient expression. In another embodiment, when using a plant cell, the expression of the fusion protein is either a transient or stable expression.

As used herein, the term "transient expression" relates to the temporary expression of genes that are expressed for a short time after a nucleic acid, most frequently plasmid DNA encoding an expression cassette, has been introduced into the host cells or plants.

As used herein, the term "stable expression" relates to expression of genes that are expressed continuously in time after a nucleic acid, most frequently plasmid DNA encoding an expression cassette, has been introduced into the host cells' genome (nuclear or plastid integration). In stably transfected cells, the foreign gene becomes part of the genome and is therefore replicated.

Both transient and stable expression could be induced by an "inducible promoter". These promoters selectively express an operably linked DNA sequence following to the presence of an endogenous or exogenous stimulus or in response to chemical, environmental, hormonal, and/or developmental signals. These regulatory element are, without limitation, sensitive to ethanol, heat, light, stress, jasmone, salicylic acid, phytohormones, salt, flooding or drought, as reviewed by Abdel-Ghany et al (2015) and discussed in US 10344290 B2, both of which are incorporated herein by reference.. Inducible promotors including, but not limited to, synthetic components discuss in Ali et al (2019) , the content of which is incorporated herein by reference.

The genus *Nicotiana* encompasses tobacco plants. Tobacco plants or plant cells have already been tested to produce recombinant immunotherapeutic binder-toxin fusion proteins composed of a small sFv fragment linked to a protein toxin with a stable linker (Francisco et al. (1997), and US6140075A.

Another example of protein fusion is the transient production in *Nicotiana benthamiana* of the human immunocytokine IL2 recombinantly fused to a scFv-Fc via a non-cleavable linker (Marusic et al. (2016). However, the production of an antibody linked to a highly potent protein toxin via a cleavable linker has never been disclosed in a plant system.

According to one further embodiment of the invention, the plant cell is at least one selected from the group consisting of:
- *Nicotiana tabacum* cv. BY2,
- *Nicotiana tabacum* NT-1,
- *Arabidopsis thaliana,*
- *Daucus carota, and*/*or*
- *Oyrza sativa.*

*Nicotiana tabacum* cv. BY2 aka Tobacco BY-2 cells and cv. *Nicotiana tabacum* 1 (NT-1, a sibling of BY-2) are nongreen, fast growing plant cells which can multiply their numbers up to 100-fold within one week in adequate culture medium and good culture conditions. This cultivar of tobacco is kept as a cell culture and more specifically as cell suspension culture (a specialized population of cells growing in liquid medium, they are raised by scientists in order to study a specific biological property of a plant cell). In cell suspension cultures, each of the cells is floating independently or at most only in short chains in a culture medium. Each of the cells has similar properties to the others.

The model plant system is comparable to HeLa cells for human research. Because the organism is relatively simple and predictable it makes the study of biological processes easier, and can be an intermediate step towards understanding more complex organisms. They are used by plant physiologists and molecular biologists as a model organism, and also used as model systems for higher plants because of their relatively high homogeneity and high growth rate, featuring still general behaviour of plant cell. The diversity of cell types within any part of a naturally grown plant (in vivo) makes it very difficult to investigate and understand some general biochemical phenomena of living plant cells. The transport of a solute in or out of the cell, for example, is difficult to study because the specialized cells in a multicellular organism behave differently. Cell suspension cultures such as tobacco BY-2 provide good model systems for these studies at the level of a single cell and its compartments because tobacco BY-2 cells behave very similarly to one another. The influence of neighboring cells behavior is in the suspension is not as important as it would be in an intact plant. As a result any changes observed after a stimulus is applied can be statistically correlated and it could be decided if these changes are reactions to the stimulus or just merely coincidental. BY-2 and NT-1 cells are relatively well understood and often used in research, including the expression of heterologous proteins, in particular antibodies (Hellwig et al (2004). Such methods are disclosed in Häkkinen et al. (2018), the content of which is incorporated herein by reference.

Torres (1989) discusses methods to establish Carrot Cell Suspension Cultures (*Daucus carota*)*.* Shaaltiel et al (2007) discuss the production of enzymes using a carrot cell based expression system. The content of these articles is incorporated herein by reference. *Daucus carota* and *Oryza sativa* are also discussed as suitable plant-cell based expressions systems in Santos et al (2016), the content of which is incorporated herein by reference. The Production of recombinant proteins in *Nicotiana tabacum, Arabidopsis thaliana, Oryza sativa* is disclosed in Plasson et al (2009), the content of which is incorporated herein by reference.

Generally, the present invention can be practiced with any plant variety for which cells of the plant can be transformed with an DNA construct suitable for expression of a foreign polypeptide and cultured under standard plant cell culture conditions. Plant cells suspension or plant tissues culture is preferred, although callus culture or other conventional plant cell culture methods may be used.

According to one other embodiment of the invention, the plant is *Nicotiana benthamiana.* The production of antibodies in Nicotiana plants is for example disclosed in Daniell et al. (2001), the content of which is incorporated herein by reference.

Other plants or plant cells that can be used in the context of the present invention include, but are not limited to, lettuce (*Lactuca spp.*)*,* spinach (*Spinacia oleracea*)*,* and Arabidopsis *(Arabidopsis spp).*

According to one further embodiment of the invention, step (i) of contacting a plant cell or a whole plant with a nucleic acid construct involves the use of a an "expression vector" alone or mediated by *Agrobacterium tumefaciens,* a vector derived therefrom, or particle bombardement/biolistic.

As used herein, the term "expression vector" relates to a vectors such as plasmids, viruses, bacteriophage, integrable DNA fragments, and other vehicles, which enable to introduce nucleic acid construct into a plant or plant cell. Expression vectors useful in the present methods are well known in the art. The term "vector" means a nucleic acid molecule, such as a plasmid, comprising regulatory elements and a site for introducing nucleic acid construct.

In some embodiment, vectors can be based on, but not limited to, full viral system, deconstructed virus, or non-viral element. The Geneware (platform described in U.S. Pat. No. 7,939,318) and MagnIcon, respectively, are the best known full viral system and deconstructed virus system, described in Altman et al (2011), the content of which is incorporated herein by reference.

Usually the used viral vectors could be classified both into RNA or DNA based vectors. As example, RNA vectors could include, without limitation, Tobamovirus (e.g. Tobacco Mosaic Virus (TMV)), cowpea mosaic virus (CMV), potexvirus (e.g. potato virus X (PVX)) or Tobravirus (e.g.tobacco rattle virus (TRV)). DNA vectors have often associated to Caulimovirus and gemini-virus who include mastrevirus, curtovirus, topocuvirus, and begomovirus. As example Bean Yellow dwarf (BeYDV) and tobacco yellow dwarf virus both from mastrevirus, have been widely used.

As an alternative, vectors can be based on non-viral elements and include for example 5' and 3' untranslated region that are synthetic (Peyret et al., 2019) or coming from other proteins (Diamos, et al., 2016). The content of these two documents is incorporated by reference herein.

It is important to note here that vectors could be replicative or not replicative. Also the replicability of the vector could be induced once activation of regulatory element as for In Plant Activation (Impact) System describe by Dugdale et al. (2013), the content of which is incorporated by reference herein.

Agrobacterium transfection is for example described in Mayo et al (2006), the content of which is incorporated by reference herein. TMV transfection is for example described in Hussain Shah et al (2013), the content of which is incorporated by reference herein. Particle bombardment/biolistic are for example disclosed in Kikkert et al (2005), the content of which is incorporated by reference herein.

It is important to stress that the advantages of the method according to the invention are not restricted to embodiments where tobacco plants or cell lines are used. For example, the fact that the conjugates can be expressed in plants because the cleavable liker or cleavable domain is left intact by the plant or plant cells is a universal principle for plants that allows the expression of the conjugates without harming the plants or plant cells. Hence, the concept of the present invention is applicable, and enabled in all plants or plant cells that can be used for recombinant expression.

Nucleic acids can be expressed in plants or plant cells under the control of a suitable operably linked promoter that is capable of expression in a given plant or plant cell. Any conventional method can be employed for plant cell transformation, culture, and regeneration, including, but not limited to, those described in the Examples below.

For the transformation of the nuclear genome, conventional techniques may be used. All known means for transiently or stably introducing foreign DNA into plant cells may be used, for example Ti plasmids, Ri plasmids, plant virus vectors, electroporation, protoplast fusion, particle gun bombardment, or penetration of DNA into cells such as pollen, microspore, seed and immature embryo. Viral vectors such as the Gemini viruses or the satellite viruses may also be used as introducing means. *Agrobacterium tumefaciens* and *rhizogenes* constitute the preferred means to introduce expression vectors. In this case, the sequence of the invention is introduced into an appropriate vector with all the necessary regulatory sequences such as promoters, terminators and the like, as well as, in the case of stable plant generation, any sequence necessary for selecting the transformants which have integrated the heterologous sequences. In particular case of induced expression, the sequence of the invention is introduced into vector containing inducible promotor and all necessary regulatory sequences.

The introduction of a nucleic acid molecule(s) into the plant cell can be carried out in a transient or stable manner either by transformation of the nuclear genome, or by transformation of the chloroplast genome of the plant cell, or by transformation of the mitochondrial genome.

The transformation of the nuclear genome of the plant cell is often carried out using the targeting signals mentioned above and which determine the cellular compartment where the expression and/or accumulation of the protein will occur.

The targeting sequences can, besides the peptide, also comprise an endoplasmic retention signal, consisting of the KDEL, SEKDEL or HEKDEL peptides. These signals normally exist at the C-terminal end of the protein and remain on the mature protein.

According to one embodiment of the invention, the peptide linker or the cleavable domain in the protoxin is specifically or non-specifically cleavable by an enzyme expressed by a mammalian cell, or an enzyme that is produced by a mammalian host.

Examples of such enzymes and their cleavage sites are shown in the following table (see also Choi et al (2012), the content of which in incorporated by reference herein. Reference is made, in this table, to the "Merops" database for more enabling information as regards the respective enzymes. https://www.ebi.ac.uk/merops/index.shtml.

| **Class** | **Enzyme class** | **example** | **cleavage sequence (one letter code)** | **reference** |
|---|---|---|---|---|
| | | | **general motif (examples only) X can be any naturally proteinogenic amino acid** | |
| "Linker class 1" Endosomal and/or Lysosomal Cleavage site | Proprotein convertase subtilisin/kexin family | Furin | RXR/KR_{↓}S/A/G/Nxxx | merops 508.071 |
| | Cathepsins | Cathepsin B | xxF/xV/R/G/L/S/A_{↓} A/F/Lxxx | merops C01.060 |
| | | Cathepsin E | xxxL/F_{↓}Vxxx | merops A01.010 |
| | | Cathepsin D | xxxL/F_{↓}xxxx | merops A01.009 |
| | | Cathepsin L | xxL/V/F/IR/K_{↓}S/A/Gxxx | merops C01.032 |
| | | Cathepsin K | xK/R/GF/L/I/V/Px_{↓}xxxx | merops C01.036 |
| | | Cathepsin C | xSxE/S_{↓}xxxG/R | merops C01.070 |
| | Granzyme | Granzyme B | V/IxxD_{↓}xxxx | merops 501.010 |
| "Linker class 2" Cytosolic cleavage site | Caspases | Caspase 3 | DxxD_{↓}A/G/S/Txxx Or xxxx_{↓}GGFV | merops C14.003 |
| | | Caspase 8 | D/LxxD_{↓}G/S/Axxx | merops C14.009 |
| | Kallikereins (hK) | hK 1 | xxF/IR/Y_{↓}R/SxGx | merops 501.160 |
| | | hK 2 | G/K/AxxR_{↓}xxxG/S/T | merops 501.161 |
| | | hK3 | S/IS/QxY/Q/R_{↓}SSxx | |
| | | hK10 | No determined | |
| "Linker class 3" Cell surface cleavage site | Matrix metallo proteases | MMP2 | xP/Axx_{↓}L/Ixxx | merops M10.003 |
| | | MMP1 | xP/Axx_{↓}L/Ixx | merops M10.001 |
| | | MMP3 | xxxR/N/G_{↓}L/Kxx | merops 501.072 |
| | | MMP7 | xPA/G/Lx_{↓}Lxxx | merops M10.005 |
| | | MMP8 | GP/A/Sxx_{↓}Lxxx | merops M10.002 |
| | | MMP9 | GP/Axx_{↓}Lxxx | merops M10.004 |
| | | | P2 is preferably a L | |
| | | | P1 is preferably a G | |
| | | MMP12 | GP/A/GL/A/Gx_{↓}Lxxx | merops M10.009 |
| | | MMP14 | xPxx_{↓}Lxxx | merops M10.014 |
| | Matriptase | Matriptase 2 | xxxR_{↓}k/G/Rxxx | merops 501.308 |
| | | Matriptase 1 | xxxR_{↓}K/V/A/RVxx | merops 501.302 |
| | tissue-type plasminogen activator | Urokinase type plasminogen activator (uPA) | xSG/SR/K_{↓}xR/Vxx | merops 501.231 |

The cleavage site is described from the cleavage site point (represented by _{↓}). The letter x refer to all amino acids. When there are several preferential amino acid, there are separated by a slash (/).

Such enzyme is preferably a protease. In one embodiment, said peptide linker is not cleavable by a plant enzyme.

Furin is an enzyme which belongs to the subtilisin-like proprotein convertase family, and cleaves proteins C-terminally of the canonic basic amino acid sequence motif Arg-X-Arg/Lys-Arg (RX(R/K)R), wherein X can be any naturally proteinogenic amino acid. Said motif is called a furin cleavage site herein. Preferably, the sequence thereof is HRRRKRSLDTS.

Cathepsins are proteases found in all animals as well as other organisms. Most of the members become activated at the low pH found in lysosomes. Cathepsin B is capable of cleaving a peptide sequence which comprises the dipeptide motif Val-Ala (VA). Said motif is called a Cathepsin B cleavage site herein. The skilled artisan finds sufficient enabling information on cathepsins and their cleavage sites in Turk el al (2012), the content of which is incorporated herein by reference.

Granzyme B is a serine protease which cleaves at unique tetrapeptide sequences. More than 580 of such tetrapeptide cleavage sites exist (Wee et al. (2011)). The tetrapeptide Ile-Glu-Pro-Asp (IEPD) was identified as the optimal tetrapeptide cleavage sequence *in vitro.* However, emerging data on granzyme B substrates suggest that the *in vivo* cleavage specificities are far more diverse, with numerous substrates possessing cleavage specificities extending beyond the tetrapeptide sequence (VanDamme et al. (2009)).

The native Granzyme B proenzyme is activated by cleavage with Cathepsin C (dipeptidyl peptidase I), at the cleavage site havening the following sequence: DAGE'IIGG. In a such way, the activated Granzyme B enzyme has an N-terminus starting with IIGGHE, and shown herein as SEQ ID NO: 1. This is hence the N-truncated sequence of the activated enzyme that is used according to one embodiment of the invention.

Caspases (cysteine-aspartic proteases, cysteine aspartases or cysteine-dependent aspartate-directed proteases) are a family of protease enzymes playing essential roles in programmed cell death. Over 1500 caspase substrates have been discovered in the human proteome. The general cleavage motif is DXXD-A/G/S/T, wherein X can be any naturally proteinogenic amino acid. The skilled artisan finds sufficient enabling information on caspases and their cleavage sites in Kumar el al (2014), the content of which is incorporated herein by reference.

Matrix metalloproteinases (MMPs), also known as matrixins, are calcium-dependent zinc-containing endopeptidases; other family members are adamalysins, serralysins, and astacins. Collectively, these enzymes are capable of degrading all kinds of extracellular matrix proteins, but also can process a number of bioactive molecules. The skilled artisan finds sufficient enabling information on Matrix Metallo Proteases and their cleavage sites in Eckard el al (2016), the content of which is incorporated herein by reference.

Generally, the skilled artisan is capable, by routine considerations and literature referral, to select specific cleavage sites that match with the respective mammalian enzyme, to control target specific release of the protein toxin or protoxin. General guidelines to find these cleavage sites are e.g. disclosed in Rawlings (2016).

According to one embodiment of the invention, the peptide linker or the cleavable domain in the protoxin is not cleavable by an enzyme expressed by a plant cell, or an enzyme that is produced by a plant host. The skilled person has a bunch of routine methods at hand to check whether this condition is met. See e.g., Wilbers et al (2016).

According to one embodiment of the invention, at least one protein toxin or protoxin is an enzyme. Because protein toxins most often act as enzymes, one toxin molecule can work on many substrate molecules, thus having a multiplexing toxicity effect on the cell - contrary to nonenzymatic toxins, which usually only work stoichiometrically with regard to the target structure.

According to one embodiment of the invention, the protein toxin is at least one of the group selected from
(1) cell death inducing proteins ("toxin class 1")
(2) protein synthesis inhibitors ("toxin class 2")
(3) membrane perturbating proteins ("toxin class 3")
(4) cell division inhibiting proteins ("toxin class 4")

Cell death inducing proteins consist, without limitation, to protein directly or indirectly acting on apoptosis pathway and/or affecting nucleic components. Cell death inducing protein target apoptosis mediated-protein, RNA and DNA, in all its formats, according to proteolytic and/or nucleolytic activities.

The class of protein synthesis inhibitors comprises proteins preventing proper operation of the ribosome. This class is mainly represented without limitation, by the group of ADP-ribosylating protein. These proteins catalyzed the ADP-ribosylation of the mammalian elongation factor 2, leading to its inactivation and blockage of the ribosome.

Membrane perturbating proteins include, but are not limited to, the group of pore-forming protein. This group of protein inserts pore into the plasma membrane allowing the free passage of electrolytes and other small molecules to disrupt the membrane integrity leading to cell lysis.

Cell division inhibiting protein is a group of protein interrupting the cell cycle by acting on element of the cytoskeleton (microtubule, tubulin, actin) and/or on protein regulating the cell cycle progression. The inhibition of microtubule dynamic and alteration of cyclin dependent protein inducing mitotic arrest and lead to cell removal.

According to one embodiment of the invention, the protein toxin or protoxin is a de-immunized variant of a native protein toxin. Recombinant methods to de-immunize protein toxins by sequence modification are disclosed, e.g., in Schmohl et al. (2015), or Grinberg and Benhar (2017), the content of which is incorporated by reference herein.

According to one embodiment, at least one protein toxin is a mammalian toxin, preferably selected from the group of Granzymes, more preferably Granzyme B, or a fragment thereof that retains the toxic activity of said protein toxin.

Granzymes are serine proteases released by cytoplasmic granules within cytotoxic T cells and natural killer (NK) cells. They induce programmed cell death (apoptosis) in the target cell, thus eliminating cells that have become cancerous or are infected with viruses or bacteria. Granzymes also kill bacteria and inhibit viral replication. In NK cells and T cells, granzymes are packaged in cytotoxic granules with perforin. Granzymes can also be detected in the rough endoplasmic reticulum, golgi complex, and the trans-golgi reticulum. The contents of the cytotoxic granules function to permit entry of the granzymes into the target cell cytosol. The granules are released into an immune synapse formed with a target cell, where perforin mediates the delivery of the granzymes into endosomes in the target cell, and finally into the target cell cytosol. Granzymes are part of the serine esterase family.

Granzyme B has both a cell death inducing effect and also inhibits cell division (Weidle et al (2014). Granzyme B cytolytic proteins induce apoptosis after release from the endosome. In addition, Granzyme B can cleave further death-substrates such as poly(ADP ribose)polymerase, DNA-dependent protein kinase, components of the cytoskeleton and the nuclear mitotic apparatus as well as proteins involved in stress response and cellular homeostasis.

Granzyme B activates apoptosis by activating caspases (especially caspase-3), which cleaves many substrates, including caspase-activated DNase to execute cell death. Granzyme B also cleaves the protein Bid, which recruits the proteins Bax and Bak to change the membrane permeability of the mitochondria, causing the release of cytochrome c (which is one of the parts needed to activate caspase-9 via the apoptosome), Smac/Diablo and Omi/HtrA2 (which suppress the inhibitor of apoptosis proteins (IAPs)), among other proteins. Granzyme B also cleaves many of the proteins responsible for apoptosis in the absence of caspase activity. The other granzymes activate cell death by caspase-dependent and caspase-independent mechanisms.

In addition to killing their target cells, granzymes can target and kill intracellular pathogens. Granzyme B cleaves viral proteins to inhibit viral activation and replication. The granzymes bind directly to the nucleic acids DNA and RNA; this enhances their cleavage of nucleic acid binding proteins.

In one embodiment, said protein toxin or protoxin is not toxic to plants or plant cells. The skilled person has a bunch of routine methods at hand to check whether this condition is met. See e.g., Klaine and Lewis (1995) for an overview, the content of which is incorporated by reference herein.

According to another aspect of the invention, a binder-toxin fusion protein produced with a method according to the above description is provided. In further embodiments, such binder-toxin fusion protein may have all the structural or functional limitations as set forth in the above description. This includes, in particular, the format of the protein binder, the linker or cleavage sites, and the specific toxin.

According to another aspect of the invention, a binder-toxin fusion protein comprising at least:
a) one protein binder selected
b) optionally, a peptide linker, and
c) at least one protein toxin or protein protoxin
is provided, wherein the binder-toxin fusion protein is encoded by a nucleic acid construct comprising in operational linkage at least the following
A) at least one polynucleotide encoding for the protein binder, or a target binding chain or domain thereof, and either
   B1) a polynucleotide encoding for a cleavable peptide linker and a polynucleotide encoding for a protein toxin, or
   B2) a polynucleotide encoding for a protein protoxin, which protoxin comprises a cleavable domain for activation thereof.

Again, in further embodiments, such binder-toxin fusion protein may have all the structural or functional limitations as set forth in the above description. This includes, in particular, the format of the protein binder, the linker or cleavage sites, and the specific toxin.

According to one embodiment of the invention, said protein comprises at least one plant-specific *N*-glycan. N-glycans are glycans that are linked to the amide group of asparagine (Asn) residues in a protein, mostly in an Asn-X-Thr or Asn-X-Ser (NXT or NXS) motif, where X is any amino acid except proline. Typical plant-specific *N*-glycans are disclosed in Gomord et al. (2010), and differ significantly from mammalian N-glycan patterns. See also Figure 7.

According to one embodiment of the invention, the protein binder binds to human CD20. In one embodiment, the protein binder is an antibody, an antibody fragment or derivative retaining target binding capacity, or an antibody mimetic.

In one embodiment, the binder comprises at least one of
a) a set comprising the 3 heavy chain CDRs and the 3 light chain CDRs as comprised in Rituximab (C2B8)
b) a heavy chain CDR/light chain CDR set of a), with the proviso that at least one of the CDRs has up to 3 amino acid substitutions relative to the respective CDR as specified in a),while maintaining its capability to bind to human CD20,
c) a heavy chain CDR/light chain CDR combination of a), with the proviso that at least one of the CDRs has a sequence identity of ≥66 % relative to the respective CDR as specified in a), while maintaining its capability to bind to human CD20.
wherein the CDRs are embedded in a suitable protein framework so as to be capable to bind to human CD20.

Rituximab (also known and C2B8) and the different patents disclosing its sequence, are enclosed in Storz U (2014), the content of which is incorporated herein by reference.

The full length sequences of Rituximab are shown herein as SEQ ID NOs 4 (heavy chain) and 5 (light chain). The variable domains of Rituximab are for example, disclosed in claim 1 and Figures 4 and 5 of EP2000149B1, the content of which is incorporated by reference herein.

Sequences that correspond to the CDRs of Rituximab are for example disclosed in SEQ ID NOs 9 - 14 shown herein below.

In some embodiments, at least one of the CDRs has a sequence identity of ≥67, preferably ≥68, more preferably any one of ≥69, ≥70, ≥71, ≥72, ≥73, ≥74, ≥75, ≥76, ≥77, ≥78, ≥79, ≥80, ≥81, ≥82, ≥83, ≥84, ≥85, ≥86, ≥87, ≥88, ≥89, ≥90, ≥91, ≥92, ≥93, ≥94, ≥95, ≥96, ≥97, ≥98 or most preferably ≥99 % sequence identity relative to the respective CDRs.

In another embodiment, at least one of the CDRs has been modified by affinity maturation or other modifications, resulting in a sequence modification compared to the sequences disclosed above.

In some embodiments, at least one of the CDRs has up to 2, and preferably 1 amino acid substitutions relative to the respective CDR as specified in a) or b).

In one embodiment, the binder comprises at least one of
a) the heavy chain/light chain variable domain pair as comprised in Rituximab (C2B8)
b) the heavy chain/light chain variable domain pair of a), with the proviso that at least one of the domains has a sequence identity of ≥80 % relative to a)
c) the heavy chain/light chain variable domain sequence pair of a), with the proviso that at least one of the domains has up to 10 amino acid substitutions relative to a), respectively,
while maintaining its capability to bind to human CD20.

In some embodiments, at least one of the domains has a sequence identity of ≥81, preferably ≥82, more preferably ≥83, ≥84, ≥85, ≥86, ≥87, ≥88, ≥89, ≥90, ≥91, ≥92, ≥93, ≥94, ≥95, ≥96, ≥97, ≥98 or most preferably ≥99 % relative to the heavy chain/light chain variable domain pair of Rituximab (C2B8).

In some embodiments, at least one of the domains has up to 9, preferably up to 8, more preferably up to 7, 6, 5, 4, 3 or 2 and most preferably up to 1 amino acid substitutions relative to the heavy chain/light chain variable domain pair of Rituximab (C2B8)

According to some embodiments of the invention, at least one amino acid substitution in the single chain diabody is a conservative amino acid substitution.

In one embodiment, the protein binder
- has a target binding affinity of ≥50 % to human CD20, compared to one of the protein binders defined above, and/or
- competes for binding to human CD20 with one of the protein binders defined above.

As used herein, the term "target binding affinity" refers to the affinity of a binding molecule according to the invention, to its target, and is expressed numerically using "KD" values. In general, a higher KD value corresponds to a weaker binding. In some embodiments, the "KD" is measured by a radiolabeled antigen binding assay (MA) or surface plasmon resonance (SPR) assays, using, e.g., a BIAcore^{™}-2000 or a BIAcore^{™}-3000. In certain embodiments, an "on-rate" or "rate of association" or "association rate" or "kon" and an "off-rate" or "rate of dissociation" or "dissociation rate" or "koff' are also determined with the surface plasmon resonance (SPR) technique. In additional embodiments, the "KD", "kon", and "koff" are measured using the Octet^{®} Systems.

As used herein, the term "competes for binding" is used in reference to one of the antibodies defined by the sequences as above, meaning that the actual antibody as an activity which binds to the same target, or target epitope or domain or subdomain, as does said sequence defined antibody, and is a variant of the latter. The efficiency (e.g., kinetics or thermodynamics) of binding may be the same as or greater than or less than the efficiency of the latter. For example, the equilibrium binding constant for binding to the substrate may be different for the two antibodies.

As used herein, the term "maintaining the capability to bind to a given target" means, for example, that the respective variant has a target binding affinity of ≥50 % compared to that of the non-modified peptide.

In this context, a "conservative amino acid substitution", as used herein, has a smaller effect on antibody function than a non-conservative substitution. Although there are many ways to classify amino acids, they are often sorted into six main groups on the basis of their structure and the general chemical characteristics of their R groups.

In some embodiments, a "conservative amino acid substitution" is one in which the amino acid residue is replaced with an amino acid residue having a similar side chain. For example, families of amino acid residues having similar side chains have been defined in the art. These families include amino acids with
- basic side chains (e.g., lysine, arginine, histidine),
- acidic side chains (e.g., aspartic acid, glutamic acid),
- uncharged polar side chains (e.g., glycine, asparagine, glutamine, serine, threonine, tyrosine, cysteine),
- nonpolar side chains (e.g., alanine, valine, leucine, isoleucine, proline, phenylalanine, methionine, tryptophan),
- beta-branched side chains (e.g., threonine, valine, isoleucine) and
- aromatic side chains (e.g., tyrosine, phenylalanine, tryptophan, histidine).

Other conserved amino acid substitutions can also occur across amino acid side chain families, such as when substituting an asparagine for aspartic acid in order to modify the charge of a peptide. Conservative changes can further include substitution of chemically homologous non-natural amino acids (i.e. a synthetic non-natural hydrophobic amino acid in place of leucine, a synthetic non-natural aromatic amino acid in place of tryptophan).

"Percentage of sequence identity" is determined by comparing two optimally aligned sequences over a comparison window, wherein the portion of the polynucleotide sequence in the comparison window may comprise additions or deletions (*i.e.,* gaps) as compared to the reference sequence (*e.g.,* a polypeptide), which does not comprise additions or deletions, for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

In further embodiments, the binder-toxin fusion protein has one of the following structures:
- (scFv-FC)-(cleavage site)-toxin/protoxin (dimer)
- tetramer of two HC and two LC-(cleavage site)-toxin/protoxin
- tetramer of two LC and two HC-(cleavage site)-toxin/protoxin

Such structures are for example shown in Figure 9, wherein CS means cleavage site, and Tox means toxin/protoxin. HC stands for Heavy chain of an IgG antibody, LC stands for Light chain of an IgG antibody. scFv-FC stands for a scFv-FC construct.

In further embodiments, the binder-toxin fusion protein has one of the following structures (N->C-orientation):
- (scFv-FC)-CS-toxin (dimer) (->toxin linked to C-terminus of FC region) (see Fig. 9A)
- tetramer of two HC and two LC-CS- toxin (->toxin linked to C-terminus of LC) (see Fig. 9B)
- tetramer of two LC and two HC-CS- toxin (->toxin linked to C-terminus of HC) (see Fig. 9C)
- toxin-CS-(scFv-FC) (dimer) (->toxin linked to N-terminus of scFv) (see Fig. 9D)
- tetramer of two HC and two toxin-CS-LC (->toxin linked to N-terminus of LC) (see Fig. 9E)
- tetramer of two LC and two toxin-CS-HC (->toxin linked to N-terminus of HC) (see Fig. 9F)
- toxin-CS-(-FC-scFv) (dimer) (->toxin linked to N-terminus of FC) (see Fig. 9G)

Therein, CS stands for "cleavage site", which is in one embodiment a furin cleavage site.

In one embodiment, the toxin is GranzymeB.

In further embodiments, the binder-toxin fusion protein comprises at least one of one of

According to another embodiment, the binder-toxin fusion protein comprises at least one of one of
- the amino acid sequence as set forth in SEQ ID NO: 2 or SEQ ID NO: 17
- two amino acid sequences as set forth in SEQ ID NO: 4 (HC of C2B8) and two amino acid sequences as set forth in SEQ ID NO: 6 (LC of C2B8-FCS-Granzyme B)
- two amino acid sequences as set forth in SEQ ID NO: 7 (HC of C2B8-FCS-Granzyme B) and two amino acid sequences as set forth in SEQ ID NO: 5 (LC of C2B8)

As regards the first option, the binder-toxin fusion protein may comprise two of the said sequences, associated to one another by means of at least one disulfide bond.

As regards the second and third option, the binder-toxin fusion protein may comprise two such pairs, conjugated to one on the by a set of disulfide bonds.

According to another aspect of the invention, a pharmaceutical composition is provided which comprises at least the fusion protein according to any the above description, and optionally one or more pharmaceutically acceptable excipients.

According to another aspect of the invention, a combination is provided which comprises (i) the fusion protein according to the above description or the pharmaceutical composition according the above description and (ii) one or more therapeutically active compounds.

According to further embodiments of the invention, the binder-toxin fusion protein according to the above description, or the composition according to the above description, or the combination according to the above description, is provided for (the manufacture of a medicament for) use in the treatment of a human or animal subject
- suffering from,
- being at risk of developing, and/or
- being diagnosed for,
developing a neoplastic disease, or for the prevention of such condition.

According to another aspect of the invention, a method for treating a human or animal subject
- suffering from,
- being at risk of developing, and/or
- being diagnosed for
developing a neoplastic disease, or for the prevention of such condition is provided, said method comprising the administration of a therapeutically effective amount of the binder-toxin fusion protein according to the above description, or the composition according to the above description, or the combination according to the above description.

It is in this respect important to stress that N-Glycans produced by plants are markedly different from those produced, e.g., in mammals. In particular, N-Glycans produced by tobacco plants have
- a Fucose residue conjugated to the proximal N-Acetyl-Glucosamine residue via a α3 glycosidic link (instead of α6 as in mammals)
- a Xylose residue conjugated to the proximal Mannose residue via a β2 glycosidic link
- two distal N-Acetyl-Glucosamine residues, each of which carry a Fucose residue via a α3 glycosidic link, and a Galactose residue via a β3 glycosidic link (instead of a neuraminic acid in mammals).

On the other hand, proteins recombinantly expressed in e.g. algae often lack any kind of glycosylation. Algae are however capable of expressing IgG shaped antibodies, or antibody fragments having a one or more disulfide bridges.

Hence, a binder-toxin conjugate produced by the novel method as discussed above has also novel structural features over a binder-toxin conjugate produced in a different expression system. See for example Figure 7 for an illustration of an N-Glycan produced by tobacco plants.

### Examples

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

All amino acid sequences disclosed herein are shown from N-terminus to C-terminus; all nucleic acid sequences disclosed herein are shown 5'->3'.

### Materials and Methods

### Genetic construct

Full length Rituximab HC and LC sequences have been used to develop mAb based binder-toxin fusion proteins. Variable parts sequences of the heavy and light chains of rituximab sequences have been assembled in a single chain scFv and fused to a human IgG1 Fc part sequence. A human furin cleavage sequence was then used to fuse the human Granzyme B sequence at the C-terminal part of the LC or the HC of the full-length rituximab or to the C-terminal part of the scFv-Fc to obtain HC + LC-FCS-Granzyme B, HC-FCS-Granzyme B + LC and scFv-c-FCS-Granzyme B fusion proteins sequences. Another binder-toxin fusion protein was realized with scFv-Fc part linked to Granzyme B without cleavage site to obtain scFv-Fc- Granzyme B. These sequences were produced by gene synthesis flanked with XbaI and IsceI.

The backbone of the pPZP200 binary plasmid was used to construct a new binary plasmid pPZP-ATB containing consecutively a *nptII* kanamycin resistance cassette, one (scFv-Fc format) or two (mAb format) gene(s) of interest expression cassette(s) and a GFP expression cassette. The ORFs coding for rituximab HC and LC, LC-FCS-GB, HC-FCS-GB, scFv-Fc-FCS-GB, scFv-Fc-GB were introduced into the appropriate binary plasmids between the Cauliflower Mosaic Virus p35S promotor (p35S) and the Agrobacterium Nopaline Synthase terminator (tNOS) using XbaI and IsceI. A version of the pPZP-ATB binary plasmid including a tomato bushy stunt virus p19 gene expression cassette between the gene of interests and the GFP cassettes was also generated.

### Transient Expression in Nicotiana benthamiana plant leaves

*Nicotiana benthaminana* grown under 16h light/8h darkness photocycle, 22 +/- 3°C. 7-8 weeks old plants leaves were transiently transformed by syringe infiltration. *Agrobacterium tumefaciens* LBA4404 (pBBRIMCS-5.virGN54D) or GV3101 (pMP90RK) harboring the pPZP-ATB-scFv-Fc-F-GB or the pPZP-ATB-scFv-Fc-F-GB-p19 binary plasmids reaching an 600 nm optical density (OD₆₀₀) around 0.8-1.0 were collected by centrifugation at 3500g for 10 min. Eventually, bacteria were adjusted to an OD₆₀₀ of 0.5 in infiltration buffer (10 mM MgCl2, 10 mM MES, 100 µM acetosyringone, pH 5,6) and the mixture was infiltrated using a needless syringe. Infiltrated regions were harvested 4 and 6 days post agroinfiltration. Entire leaves harvested 4 days post agroinfiltration were used for protein A purification.

### Expression in N. tabacum cells

*Nicotiana tabacum* plant suspension cells were grown 5 days at 130 rpm, 25°C in plant culture media as described by Nagata et al. (1992), the content of which is incorporated herein. *Agrobacterium tumefaciens* LBA4404 (pBBRIMCS-5.virGN54D) harboring the pPZP-ATB binary plasmids reaching an 600 nm optical density (OD₆₀₀) around 0.8-1.0 were collected by centrifugation at 2000g for 5 min. Plant cells and bacterial cells were then cocultivated in cocultivation media for 30 min before a 2000g 5 min centrifugation. After supernatant removal, cells were plated on solid cocultivation media for two days. In the case of transient transformation, cells were then collected and washed three times and cultivated in plant cultivation media containing Cefotaxim and Carbeniclin before being harvested for further analysis. In the case of stable transformation, after the 2 days of solid cocultivation, cells were washed and plated on plant media containing selective kanamycin and Cefotaxim and Carbeniclin antibiotics. Callus were selected 4 weeks later and subcultured on solid media or in liquid suspension cultures for subsequent analysis.

### Protein analysis: ELISA, SDS-PAGE and Westernblot

Collected leaves tissues (120 mg) were ground in 400 µL extraction buffer (250 mM Sorbitol, 60 mM Tris, Na2EDTA, 0.6% Polyclar AT, 1 mM PMSF, pH8.0 supplemented with 2 µg/mL each of protease inhibitors: Leupeptin, aprotinin, antipain, pepstatin A, Chymostatin). Homogenized tissue was centrifugated at 4°C for 40 min at 18200g. Supernatant was then recovered, froze in liquid nitrogen and stored at -20°C.

Extracted tissue were analyzed by westernblotting. Proteins were boiled for 5 min in reducing or non-reducing SDS loading buffer (80 mM Tris-HCl, pH 6.8, 2% SDS, 10% glycerol, 0.005% bromophenol blue supplemented with 1 mM PMSF and protease inhibitor cocktail: 2 µg/mL each of leupeptin, aprotinin, antipain, chymostatin and pepstatin), centrifuged for 5 min at 13 000 rpm and separated by SDS-PAGE (4-20% polyacrylamide). For Western blotting, proteins were electrotransferred onto a PVDF membrane (Biorad) using a semi-dry electrophoretic device (Biorad Trans-Blot Turbo); then, the membrane was blocked for 1 h at room temperature with 3% (w/v) non-fat milk powder in TBST buffer (50 mM Tris-HCl, 150 mM NaCl, 0.5% Tween 20, pH 7.5) and then incubated (TBS-Tween 0.1% + 0.5% non-fat dry milk) for 1 h at room temperature with HRP-conjugated antibodies against the anti-human IgG Fc specific region (A0170; Sigma-Aldrich), at a dilution of 1 : 10.000 or against human Granzyme B primary antibody (EPR20129-217; Abcam) at a dilution of 1 : 10.000. The anti-Granzyme B antibody was followed by HRP-conjugated anti-rabbit antibodies (0545; Sigma), at a dilution of 1 : 10 000. Proteins were detected by enhanced chemiluminescence (Amersham Imager 600/GE; GE Healthcare).

### Anti CD20 ELISA

For anti-CD20 conjugate specificity analysis, plant extracts were analyzed by 96 well microplate (Greiner) were coated with 100 µL 5µg/mL CD20 (AcroBiosystems) for 2h at 37°C then washed 5 times in washing buffer (TBS Tween 0,1%). Blocking was then performed with 200µL BSA 1% in TBS pH8.0 for 30 min at RT then washed 5 times. 100 µL Anti-CD20 control antibody was loaded to realize a calibration curve between 5 and 0 µg/mL and 100 µL samples were loaded on the same 96 well plates for comparison for 2h at RT then washed 5 times. 100 µL of 1/150.000 diluted detection antibody (goat anti-human HRPO, Bethyl) was loaded and incubated 1h at RT. Revelation was then performed with 100 µL TMB reaction buffer (Zentech) for 30 min and finally stopper with H₃PO₄ 1M. Enzymatic activity was then analyzed by spectrometry at 450 nm.

### Further ELISA

For specificity analysis of a conjugate specific for a non-disclosed antigen (structure expressed on the surface of human cells, and overexpressed in some cancers, called antigen X herein), a purified binder-toxin fusion protein comprising a binder against X was analysed by 96 well microplate (Greiner). The wells were coated with 50 µl of antigen X (2,5 µg/mL) for 1h at 37°C then washed 5 times with 250µL washing buffer (PBS Tween 0,1%). Blocking was then performed with 150µL hydrocasein (3.6%) in PBST for 30 min at RT then washed 5 times. 50 µL anti antigen control antibody was loaded to realize a calibration curve between 5 and 0 µg/mL and 50 µL samples were loaded on the same 96 well plates for comparison for 1h at RT then washed 5 times. 50 µL of 1/200.000 diluted detection antibody (goat anti-human HRPO, Bethyl) was loaded and incubated 1h at RT. Revelation was then performed with 50 µL TMB reaction buffer (Zentech) for 15 min and finally stop with H₃PO₄ 1M. Enzymatic activity was then analyzed by spectrometry at 450 nm. Results are shown in Fig. 4B.

### Protein A purification

Four days post agroinfiltration, leaves were collected, weighted and grinded in a blender using 2 mL of extraction buffer (250 mM Sorbitol, 60 mM Tris, Na2EDTA, 0.6% Polyclar AT, 1 mM PMSF, pH8.0 supplemented with 2 µg/mL each of protease inhibitors :Leupeptin, aprotinin, antipain, pepstatin A, Chymostatin) per gram of fresh agroinfiltrated leaves. The mixture was then filtered through a double Miracloth (Millipore) layer. The filtrate was then centrifugated at 4°C for 30 min at 20.000g. Supernatant was then loaded onto protein A resin preequilibrated with extraction buffer. Resin was then washed with 10 column volume of 60 mM Tris pH8.0 and elution was performed using 100 mM glycine pH3.0 directly buffered with 10% Tris 1M pH8.0. Enriched protein fractions were then collected and freeze in liquid nitrogen.

### In vitro cytotoxicity assay

The anti-CD20 antibody Rituximab and Rituximab based conjugates effects on target cells Raji (CD20+) and non-target cells Loucy (CD20) is evaluated by an *in vitro* cytotoxicity assay (MTT). Briefly, Raji and Loucy cell lines are cultivated and resuspended at a density of 1.10⁵ cells/50µL in FBS free culture medium and 50 µl of the cell suspension was dispatched in 96-well flat bottom plate. Then, Rituximab and conjugates comprising the latter are incubated with the cells at various concentrations for 6, 24, and 48h at 37°C, 5% CO₂. For each time point, the cytotoxicity is evaluated using a MTT kit assay (Roche). Cell viability is calculated by measuring the absorbance at 550 nm. Viability mean were calculated for triplicate assays.

### Further in vitro cytotoxicity assay

The effect of a purified binder-toxin fusion protein comprising a binder against the non-disclosed antigen X was analyzed on the viability of cell lines was assessed using the Cell Proliferation Reagent WST-1 (Merck, 5015944001). WST-1 *(4-[3-(4-Iodophenyl)-2-(4-nitrophenyl)-2H-5-tetrazolio]-1,3-benzene disulfonate)* is the substrate for mitochondrial dehydrogenases and is cleaved to formazan. The amount of formazan dye formed is directly correlated to the number of metabolically active cells in the culture.

Cells were seeded in 96 well microtiter plates at a density of 30.000 cells/well in 50 ml of growth medium. Dilutions of the binder-toxin fusion protein or buffer were prepared by adding 10 ml of binder-toxin fusion protein or buffer to 40 ml of growth medium and the mixture was incubated to the cells. Binder-toxin fusion proteins were tested in duplicate. Buffer and positive control (2% Triton and 5% DMSO) were tested in triplicate.

After 72h incubation at 37°C with 5% CO₂, 10 ml of Cell Proliferation Reagent WST-1 (Merck, 5015944001) was added to each well and the plates were incubated for an additional 4h at 37°C and 5% CO₂. The formazan dye was quantified by reading the OD of the plates at 450 nm and 690 nm for background subtraction.

To determine the % of viability of non-treated cells and cells treated with binder-toxin fusion protein and positive controls, the average OD value of the wells treated with buffer was calculated (OD₄₅₀ₙₘ-OD₆₉₀ₙₘ) and set to 100% viability. Results are shown in Fig. 4A.

### Peptide glycoform analysis

To demonstrate that binder-toxin fusion proteins made with the method according to the present invention are structurally different from binder-toxin fusion proteins made in other expression systems, like e.g. mammalian, glycoforms of the peptides EEQY**NST**YR and (**NFS**NDIMLLQLER, both comprising a bold-marked N-glycosylation site, disclosed as SEQ ID NOs 15 and 16 herein, and being comprised in some of the binder-toxin fusion proteins disclosed herein) made with different *Nicotiana benthamiana* strains were analyzed. ATB_3 and ATB_4 are wild types, ATB_22 and ATB_24 were knocked-down by RNAi for the endogenous β1,2-xylosyltransferase (XylT or XT) and α1,3-fucosyltransferase (FucT or FT) genes (for methods see Strasser et al (2008), the content of which is incorporated herein by reference.

| Strain name | ATB_3 | ATB_4 | ATB_22 | ATB_24 |
|---|---|---|---|---|
| comments | Wild type | Wild type | ΔXT/FT | ΔXT/FT |

Briefly, the samples were digested in solution. The proteins were S-alkylated with iodoacetamide and digested with Trypsin (Promega).

The digested samples were loaded on a BioBasic C18 column (BioBasic-18, 150 x 0.32 mm, 5 µm, Thermo Scientific) using 80 mM ammonium formiate buffer as the aqueous solvent. A gradient from 5% B (B: 80% ACCN) to 40% B in 45 min was applied, followed by a 15min gradient from 40% B to 90% B that facilitates elution of large peptides, at a flow rate of 6 µL/min. Detection was performed with QTOF MS (Bruker maXis 4G) equipped with the standard ESI source in positive ion, DDA mode (= switching to MSMS mode for eluting peaks). MS-scans were recorded (range: 150-2200 Da) and the 3 highest peaks were selected for fragmentation. Instrument calibration was performed using ESI calibration mixture (Agilent). The three possible glycopeptides were identified as sets of peaks consisting of the peptide moiety and the attached N-glycan varying in the number of HexNAc units, hexose, deoxyhexose and pentose residues. The theoretical masses of these glycopeptides were determined with a spread sheet using the monoisotopic masses for amino acids and monosaccharides.

Manual glycopeptide searches were made using DataAnalysis 4.0 (Bruker). For the quantification of the different glycoforms the peak areas of EICs (Extracted Ion Chromatograms) of the first four isotopic peaks

### Cleavage assays

Cleavability allowing the release of the toxin have been proved *in vitro* after addition of recombinant furin on purified scFv-Fc-FCS-GB (binder-toxin fusion protein). The reaction was performed overnight at 25 degree following addition of 25 units/ml furin (NEB P8077S) to 6 microgram of binder-toxin fusion protein into 35 µl of cleavage buffer (20 mM Hepes, Triton X-100 0,1 %, 1 mM CaCl2, pH7.5). Cleavage have been visualized by SDS Page Coomassie blue gel (4-20% polyacrylamide). A proliferation-inducing ligand (APRIL) from mouse was used as control of furin cleavage. APRIL (SRP3189 - Sigma-Aldrich, 20 µg lyophilizate protein) was resuspended into 20 µL of water containing 0.1% BSA.

### Results

In this study, we designed several recombinant binder-toxin fusion proteins composed of a binding moiety fused to a toxin by a cleavable linker and successfully expressed them in plant cells or entire plants. Rituximab (C2B8) based full-length mAb, scFv-Fc format and scFv format were used as binding moieties. Rituximab and its sequence is described in Storz (2014). The furin cleavage sequence (FCS) having the amino acid sequence HRRRKRSLDTS was used as cleavable linker. Another binder-toxin fusion protein constructed with scFv-Fc recombinantly linked to native granzyme B without cleavage sequence was also used as example of recombinant non cleavable linkers. Human Granzyme B was used as payload example. Payload position may vary on the binding moiety, in this study we exemplified fusion of the Granzyme B at the C-terminus of a full length mAb heavy or light chains.

Several recombinant binder-toxin fusion proteins based on the scFv-Fc format have been constructed: scFv-Fc-FCS-Granzyme B, scFv-Fc- Granzyme B, scFv-Fc alone was also constructed as control. Two binder-toxin fusion proteins based on full length mAb have been constructed: HC+LC-FCS-Granzyme B, HC-FCS-Granzyme B+LC. Unconjugated mAb alone was also constructed as control.

The following table summarizes the conjugates produced herein:

| **Conjugate type** | **protein binder** | **Linker** | **Cleavage sequence (AA one letter code)** | **Protein toxin** | **Sequence Ref** |
|---|---|---|---|---|---|
| scFv-Fc (control) | scFv-Fc | NA | | NA | 1 |
| scFv-Fc-FCS-GranzymeB | scFv-Fc | furin cleavage site (FCS) | HRRRKRSLDTS | Granzyme B | 2 |
| scFv-Fc-GranzymeB | scFv-Fc | No cleavage site | NA | Granzyme B | 3 |
| mAb (control) | Heavy chain + light chain | NA | NA | NA | 4 (HC) and 5 (LC) |
| HC+LC-FCS-Granzyme B | heavy chain + fused light chain | furin cleavage site (on light chain C-terminal part) | HRRRKRSLDTS | Granzyme B | 4 and 6 |
| HC-FCS-Granzyme B+LC | fused heavy chain + light chain | furin cleavage site (on heavy chain C-terminal part) | HRRRKRSLDTS | Granzyme B | 7 and 5 |
| scFv-Fc-LINK2-TOX2 | scFv-Fc | Linker of class 2 | LINK2 | TOX2 | |
| scFv-Fc-FCS-TOX2 | scFv-Fc | furin cleavage site (FCS) | RHRR | TOX2 | |
| scFv-Fc-LINK3-TOX3 | scFv-Fc | Linker of class 3 | LINK3 | TOX3 | |

The DNA sequence of each DNA construct was plant codon optimized and introduced in a pPZP-ATB agrobacterium binary transformation vector between the Cauliflower Mosaic Virus p35S promotor (p35S) and the Agrobacterium Nopaline Synthase terminator (tNOS). Approaches for codon optimizing in tobacco plants are disclosed in Rouwendal et al. (1997), the content of which is incorporated herein by reference.

The same construct was also introduced in a pPZP-ATB-p19 carrying a supplementary expression cassette for the silencing suppressor gene p19. In this example, an additional neomycin phosphotransferase II (nptII) kanamycin resistance cassette was inserted for stable clone selection. All constructs were electroporated into *Agrobacterium tumefaciens* LBA4404 or GV3101.

### Entire plant expression system

Agrobacterium strains were first used to transiently transform *Nicotiana benthamiana* by syringe agroinfiltration with a construct encoding a scFv-Fc-F-Granzyme B conjugate. Agroinfiltrated plant tissue extracts were then analyzed by western blotting using anti-human IgG Fc part antibodies (Figure 1) after 4 and 6 days post agroinfiltration (dpa). The impact of the use of *Agrobacterium tumefaciens* LBA4404 or Agrobacterium GV3101 strains, as well of the harvesting day and of p19 co-expression on the fusion protein expression was evaluated. The fusion protein is expressed and seems mainly intact after 4 days post agroinfiltation when expressed using *Agrobacterium tumefaciens* LBA4404 strain (Fig1, lane 1 &2). More degradation product seems to appear after 6 days post agroinfiltration or using GV3101 strain. Addition of the p19 suppressor of gene silencing seems to have no effect on expression when carried out by the LBA4404 (Figure 1, lane 2 & 4) compared to the GV3101 strain showing expression only in presence of the p19 (Figure 1, lane 6 & 8). Regarding the harvesting time, more degradation fragments appears after 6 days post agroinfiltration (Figure 1, lane 3, 4 & 8).

The scFv-Fc-FCS-Granzyme B fusion protein is composed of two monomeric form of 80 kDa resulting in complete dimeric form of around 160 kDa. Each monomer components have been identified by western blotting on proteins extracts after *Agrobacterium tumefaciens* LBA4404 infection as used in Figure 1 (left panel), using specific detection mAb directed against human IgG Fc part (Figure 2, left) or the human Granzyme B (Figure 2, right) in reducing (+DTT) and non-reducing (-DTT) conditions. Detection of the full size scFv-Fc-FCS-Granzyme B (Figure 2) is confirmed by the detection of a signal at about 250 kDa using anti IgG Fc part (left panel, -DTT) and by the detection of a signal at the same size (right panel, -DTT) using an anti-Granzyme B antibody. Note that the human serum IgGs of around 150 kDa also result in a signal around 250 kDa. In reducing conditions, the monomeric form is also detected by both anti IgG Fc antibody and anti-Granzyme B antibody with a signal between 70 and 100 kDa (Indicated by Δ on the left and right panel) near to the monomer expected size of 80 kDa.

Other formats of binder-toxin fusion proteins have been transiently expressed in *Nicotiana benthamiana* using *Agrobacterium tumefaciens* LBA4404, plant leaves extracts are presented Figure 3. scFv-Fc-FCS-Granzyme B, scFv-Fc-Granzyme B is detected at the expected size. Moreover, the full mAb based binder-toxin fusion protein HC-FCS-Granzyme B + LC is also detected at the expected size.

### Plant cells expression system

Agrobacterium strains were also used to transiently transform *Nicotiana tabacum* cv. Bright Yellow 2 cells (BY-2 cells). Different binder-toxin fusion proteins have been transiently expressed in *Nicotiana tabacum* cv. BY-2. The intracellular extracts were then analyzed by western blotting using a anti Fc- human IgG.

The expression of the scFv-Fc-FCS-Granzyme B identity has been confirmed in Figure 5, the dimeric full size fusion protein is detected at a size around 250 kDa by the anti-human IgG Fc part and the anti-Granzyme B antibody.

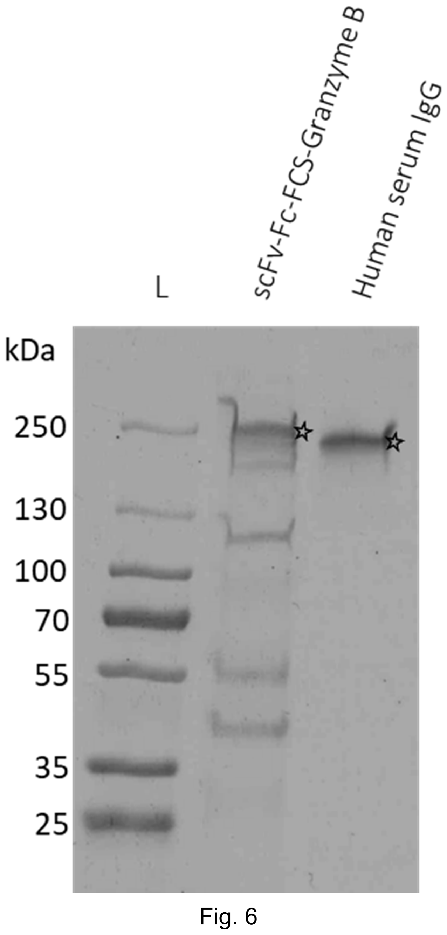

These binder-toxin fusion proteins can be stably expressed in plant suspension cells as well. As an example, we performed a *Nicotiana tabacum* cv. BY-2 plant cells stable transformation by cocultivation with agrobacterium strain. In this case, the kanamycin resistance cassette was used to select stable plant cells clones. Figure 6 shows the scFv-FCS-Granzyme B binder-toxin fusion protein presence in the stable plant suspension cells at the integral expected size indicating that this kind of molecule can be stably expressed in plant cell system.

A new binder-toxin fusion protein composed of a scFv-Fc, a furin cleavage site and a toxin among the toxin family 2 (scFv-Fc-FCS-TOX2) was also successfully expressed in plant cells (Figure 8, left panel) and in entire plant (Figure 8, right panel). TOX2 is a toxin from class 2 as disclosed herein (protein synthesis inhibition). The full size binder-toxin fusion protein is detected at its expected size which is similar to scFv-Fc-FCS-Granzyme B already presented above.

Together, these data confirm that binder-toxin fusion proteins composed of a protein binder linked via a human cleavable sequence to a cytotoxic protein payload are producible in entire plants or plant cells systems.

The binding function of the scFv-Fc-GB conjugate produced in plant cells has been assessed by anti CD20 Elisa, having an affinity comparable with that of Rituximab (data not shown). In vitro cytotoxicity assays were also performed on CD20+ cells (Raji cells). Similar or improved cytotoxicity compared to rituximab is awaited. These results suggests that the Fc part of the scFv-Fc-GB is functional and exhibits biological cytotoxicity on targeted cells.

### Peptide glycoform analysis

In Fig 10, the MS spectra of the glycosites (EEQY**NST**YR and **NFS**NDIMLLQLER) are shown (SEQ ID NO 15 and 16, comprised in some of the binder toxin fusion peptides disclosed herein.

The major glycoforms identified were complex type glycans (GnGn/GnGnXF). Other glycoforms (Man5-Man9, GnGnF, GnGnX, MMXF, Man5Gn and GnM(X)(F)) were detected as well. Table 1 lists structures and their relative proportions. All samples contained non-glycosylated peptide.

The peak heights in the MS spectra roughly reflect the molar ratios of the glycoforms (Note that more than one charge state is present per glycoform). In **Fehler! Verweisquelle konnte nicht gefunden werden.** and Table 2 the quantitation of the different glycoforms is shown (quantified by integration of EIC of the first 4 isotopic peaks). For an explanation of the abbreviations of the different glycoforms see Strasser et al (2008), the content of which is incorporated herein by reference.

| **EEQYNSTYR** | ATB_3 | ATB_4 | ATB_22 | ATB_24 |
|---|---|---|---|---|
| *glycan* | *% of total* | | | |
| not glyc | 10.23 | 14.70 | 30.84 | 2.15 |
| Man5 | 1.37 | 1.10 | 4.38 | 0.68 |
| Man6 | 0.66 | 0.69 | 0.90 | |
| Man7 | 1.37 | 1.32 | 1.94 | |
| Man8 | 0.94 | 1.22 | 1.95 | 0.94 |
| Man9 | 0.94 | 1.26 | 1.01 | |
| MM | | | 1.21 | |
| MMF | | 0.58 | | |
| MMX | | | | |
| MMXF | 1.29 | 7.03 | | |
| GnM | 0.64 | 0.44 | 4.81 | 8.49 |
| GnMF | 0.72 | 0.60 | | |
| GnMX | 0.79 | 0.65 | | |
| GnMXF | 3.40 | 6.35 | | 0.71 |
| GnGn | 7.43 | 5.56 | 45.33 | 82.49 |
| GnGnF | 5.89 | 2.90 | 0.63 | 0.83 |
| GnGnX | 8.71 | 7.14 | | 0.41 |
| GnGnXF | 48.52 | 44.45 | | |
| AGnF | 1.48 | 1.23 | | |
| Gn(FA)XF | 5.21 | 2.29 | | |
| Man4Gn | | | 2.08 | 1.86 |
| Man5Gn | | | 3.50 | 0.77 |
| AGn | 0.43 | 0.49 | 1.43 | 0.67 |

### Proportion of glycoforms in % for EEQYNSTYR

| **NFSNDIMLLQLER** | ATB_3 | ATB_4 | ATB_22 | ATB_24 |
|---|---|---|---|---|
| *glycan* | % *of total* | | | |
| not glyc | 20.53 | 23.92 | 45.24 | 15.28 |
| Man5 | | 7.25 | 2.48 | 0.42 |
| Man6 | 0.80 | 0.82 | | |
| Man7 | | 0.50 | | |
| Man8 | 0.49 | 1.39 | 1.94 | |
| Man9 | | 0.85 | | |
| MGn | | 0.85 | 1.87 | 5.27 |
| MGnF | 0.47 | 2.10 | 0.93 | 1.90 |
| MGnX | 1.01 | 0.91 | | 0.43 |
| MGnXF | 18.77 | 10.22 | | 0.79 |
| GnGn | | | 34.53 | 48.23 |
| GnGnF | | 0.63 | 2.17 | 14.57 |
| GnGnX | 0.56 | 0.85 | | 1.09 |
| GnGnXF | 35.89 | 30.80 | | 0.56 |
| Gn(FA) | | 0.90 | 10.84 | 10.98 |
| Gn(FA)XF | 21.01 | 18.01 | | 0.48 |

### Proportion of glycoforms in % for NFSNDIMLLQLER

Please note that one possible isomer is given and the used method only gives the composition of the glycans. For glycan names, the proglycan nomenclature was used (http://www.proglycan.com/protein-glycosylation-analysis/nomenclature). see also the reference document "What's your name, sugar ? - A simple abbreviation system for complex N-glycan structures"

According to this nomenclature, MGnX means for example

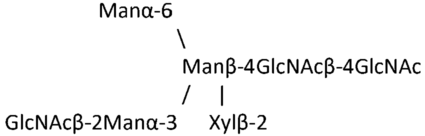

### References

- Daniell H et al., Trends Plant Sci. 2001 May; 6(5): 219-226.
- Häkkinen S et al., Front Plant Sci. 2018; 9: 45.
- Gomord V et al., (2010),. Plant Biotechnology Journal, 8: 564-587
- Storz U, MAbs. 2014 Jul-Aug;6(4):820-37
- Nagata, T et al., (1992). International Review of Cytology (Vol. 132, pp. 1-30).
- Schmohl J et al, Toxins (Basel). 2015 Oct; 7(10): 4067-4082.
- Yehudit Grinberg Y and Benhar I, Biomedicines. 2017 Jun; 5(2): 28.
- Grawunder, Ulf, and Stefan Barth, eds. Next Generation Antibody Drug Conjugates (ADCs) and Immunotoxins. Springer, 2017
- Yao, Jian, et al., International journal of molecular sciences 16.12 (2015): 28549-28565
- Yin, Jiechao, et al. Journal of biotechnology 127.3 (2007): 335-347.
- Alewine, Christine, Raffit Hassan, and Ira Pastan. The oncologist (2015): theoncologist-2014.
- Guo, Rui, et al. International journal of pharmaceutics 511.1 (2016): 538-549.
- Mathieu-Rivet, Elodie, et al. Frontiers in plant science 5 (2014): 359.
- Mohamedali, Khalid A., et al. Molecular cancer therapeutics (2013).
- Drake, Penelope M., and David Rabuka. Current opinion in chemical biology 28 (2015): 174-180.
- Peyret, H., Brown, J. K., & Lomonossoff, G. P. (2019). Plant methods, 15(1), 108.
- Diamos, A. G., Rosenthal, S. H., & Mason, H. S. (2016). Frontiers in plant science, 7, 200.
- Peters, Christina, and Stuart Brown. Bioscience reports (2015): BSR20150089.
- Parslow, Adam C., et al. Biomedicines 4.3 (2016): 14.
- Rouwendal, G.J., Mendes, O., Wolbert, E.J. et al. Plant Mol Biol (1997) 33: 989.
- Kikkert JR et al. (2005), Methods in Molecular Biology 286:61-78
- Mayo KJ et al. Nat Protoc. 2006;1(3):1105-11.
- Hussain Shah K et al, Plant Mol Biol Report. 2013; 31(6): 1529-1538.
- Gebauer and Skerra, Curr Opin Chem Biol. 2009 Jun;13(3):245-55.
- Köhler and Milstein, Nature. Bd. 256, S. 495-497
- Francisco JA., et al., Binder-toxin fusion protein chemistry 8.5 (1997): 708-713.
- Marusic C et al., Plant biotechnology journal 14.1 (2016): 240-251.
- Wee LJK et al, BMC Genomics. 2011; 12(Suppl 3): S11.
- Van Damme P et al, Mol Cell Proteomics. 2009 Feb; 8(2):258-72.
- Santos R et al., Front. Plant Sci., Front. Plant Sci., 11 March 2016
- Klaine, S. J. and M A. Lewis. Algal And Plant Toxicity Testing. 1995 Chapter 8, in Hoffman et al (eds), Handbook of Ecotoxicology. Lewis Publishers, Boca Raton, FL, 163-184, (1995).
- Turk V et al, Biochimica et Biophysica Acta (BBA) - Proteins and Proteomics, Volume 1824, Issue 1, January 2012, Pages 68-88
- Kumar S et al, PLoS One. 2014; 9(10): e110539
- Eckard U et al, Matrix Biology, Volume 49, January 2016, Pages 37-60
- Rawlings ND, Biochimie, Volume 122, March 2016, Pages 5-30
- Torres, K. C. (1989).In Tissue Culture Techniques for Horticultural Crops(pp. 161-163). Springer, Boston, MA.)
- Shaaltiel, Y et al., (2007) Plant biotechnology journal, 5(5), 579-590
- Plasson, C et al., (2009), Recombinant Proteins From Plants (pp. 145-161). Humana Press.
- Bujak E et al., Methods Mol Biol. 2014;1131:315-34
- Choi, KY, et al., Theranostics 2.2 (2012): 156.
- Wilbers, RH et al, Plant biotechnology journal, 14(8), 1695-1704
- Weidle, UH et al, Cancer Genomics-Proteomics, 11(2), 67-79
- Hellwig, S et al., Nature biotechnology, 22(11), 1415
- Strasser R et al., Plant Biotechnology Journal(2008) 6, pp. 392-402
- What's your name, sugar ? - A simple abbreviation system for complex N-glycan structures - Anon, 2007 Hussain Shah et al (2013)
- Dugdale, Benjamin, et al., The Plant Cell 25.7 (2013): 2429-2443
- Hempel F et al., PLoS One. 2011; 6(12)
- Abdel-Ghany S. E. (2015). Engineering of plants for the production of commercially important products: approaches and accomplishments. In Plant biology and biotechnology (pp. 551-577). Springer.
- Ali, S., & Kim, W. C. (2019). A fruitful decade using synthetic promoters in the improvement of transgenic plants. Frontiers in plant science, 10.
- Altman, A., & Hasegawa, P. M. (Eds.). (2011). Plant biotechnology and agriculture: prospects for the 21st century. Academic press.

### Abbreviations as used herein

HC = antibody heavy chain
LC = antibody Light chain
TOX = Toxin
CS = cleavage site
FCS = Furing cleavage site
LINK = Linker/Cleavage site
LINK2: Linker of class 2 (=cytosolic cleavage site),
LINK3: Linker of class 3 (=cell surface cleavage site)
GB = Granzyme B
TOX2: Toxin of class 2 (=protein synthesis inhibition),
TOX3: Toxin of class 3 (=membrane perturbating protein),
scFv: single chain format
scFv-FC: scFv-FC-format

### Sequences

The following sequences form part of the disclosure of the present application. A WIPO ST 25 compatible electronic sequence listing is provided with this application, too. For the avoidance of doubt, if discrepancies exist between the sequences in the following table and the electronic sequence listing, the sequences in this table shall be deemed to be the correct ones.

| **No** | **Qualifier** | **Fig** | **Sequence** |
|---|---|---|---|
| 1 | anti CD20 scFv-Fc | | QIVLSQSPAILSASPGEKVTMTCRASSSVSYIHWFQQKPGSSPKPWIYATSNLASGVPVR FSGSGSGTSYSLTISRVEAEDAATYYCQQWTSNPPTFGGGTKLEIKGGGGSGGGGSGGGG SQVQLQQPGAELVKPGASVKMSCKASGYTFTSYNMHWVKQTPGRGLEWIGAIYPGNGDTS YNQKFKGKATLTADKSSSTAYMQLSSLTSEDSAVYYCARSTYYGGDWYFNVWGAGTTVTV SAEPKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK |
| 2 | anti CD20 scFv-Fc-FCS-Granzyme B | 9A | QIVLSQSPAILSASPGEKVTMTCRASSSVSYIHWFQQKPGSSPKPWIYATSNLASGVPVR FSGSGSGTSYSLTISRVEAEDAATYYCQQWTSNPPTFGGGTKLEIKGGGGSGGGGSGGGG SQVQLQQPGAELVKPGASVKMSCKASGYTFTSYNMHWVKQTPGRGLEWIGAIYPGNGDTS YNQKFKGKATLTADKSSSTAYMQLSSLTSEDSAVYYCARSTYYGGDWYFNVWGAGTTVTV SAEPKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGKHRRRKR SLDTSIIGGHEAKPHSRPYMAYLMIWDQKSLKRCGGFLIRDDFVLTAAHCWGSSINVTLG AHNIKEQEPTQQFIPVKRPIPHPAYNPKNFSNDIMLLQLERKAKRTRAVQPLRLPSNKAQ VKPGQTCSVAGWGQTAPLGKHSHTLQEVKMTVQEDRKCESDLRHYYDSTIELCVGDPEIK KTSFKGDSGGPLVCNKVAQGIVSYGRNNGMPPRACTKVSSFVHWIKKTMKRY |
| 3 | anti CD20 scFv-Fc-Granzyme B | | QIVLSQSPAILSASPGEKVTMTCRASSSVSYIHWFQQKPGSSPKPWIYATSNLASGVPVR FSGSGSGTSYSLTISRVEAEDAATYYCQQWTSNPPTFGGGTKLEIKGGGGSGGGGSGGGG SQVQLQQPGAELVKPGASVKMSCKASGYTFTSYNMHWVKQTPGRGLEWIGAIYPGNGDTS YNQKFKGKATLTADKSSSTAYMQLSSLTSEDSAVYYCARSTYYGGDWYFNVWGAGTTVTV SAEPKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGKGEIIGG HEAKPHSRPYMAYLMIWDQKSLKRCGGFLIRDDFVLTAAHCWGSSINVTLGAHNIKEQEP TQQFIPVKRPIPHPAYNPKNFSNDIMLLQLERKAKRTRAVQPLRLPSNKAQVKPGQTCSV AGWGQTAPLGKHSHTLQEVKMTVQEDRKCESDLRHYYDSTIELCVGDPEIKKTSFKGDSG GPLVCNKVAQGIVSYGRNNGMPPRACTKVSSFVHWIKKTMKRY |
| 4 | HC of anti CD20 antibody C2B8 | 9B | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVS AASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKAEPKSCDKTHTCPPCPAPELLG GPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 5 | LC of anti CD20 | 9C | QIVLSQSPAILSASPGEKVTMTCRASSSVSYIHWFQQKPGSSPKPWIYATSNLASGVPVR FSGSGSGTSYSLTISRVEAEDAATYYCQQWTSNPPTFGGGTKLEIKRTVAAPSVFIFPPS DEQLKSGTASVVCLLNNFYPREAKVQWKVDNALQSGNSQESVTEQDSKDSTYSLSSTLTL |
| | antibody C2B8 | | SKADYEKHKVYACEVTHQGLSSPVTKSFNRGEC |
| 6 | (anti CD20 antibody C2B8)-LC-FCS-GranzymeB | 9B | QIVLSQSPAILSASPGEKVTMTCRASSSVSYIHWFQQKPGSSPKPWIYATSNLASGVPVR FSGSGSGTSYSLTISRVEAEDAATYYCQQWTSNPPTFGGGTKLEIKRTVAAPSVFIFPPS DEQLKSGTASVVCLLNNFYPREAKVQWKVDNALQSGNSQESVTEQDSKDSTYSLSSTLTL SKADYEKHKVYACEVTHQGLSSPVTKSFNRGECHRRRKRSLDTSIIGGHEAKPHSRPYMA YLMIWDQKSLKRCGGFLIRDDFVLTAAHCWGSSINVTLGAHNIKEQEPTQQFIPVKRPIP HPAYNPKNFSNDIMLLQLERKAKRTRAVQPLRLPSNKAQVKPGQTCSVAGWGQTAPLGKH SHTLQEVKMTVQEDRKCESDLRHYYDSTIELCVGDPEIKKTSFKGDSGGPLVCNKVAQGI VSYGRNNGMPPRACTKVSSFVHWIKKTMKRY |
| 7 | (anti CD20 antibody C2B8)-HC-FCS-GranzymeB | 9C | QVQLQQPGAELVKPGASVKMSCKASGYTFTSYNMHWVKQTPGRGLEWIGAIYPGNGDTSY NQKFKGKATLTADKSSSTAYMQLSSLTSEDSAVYYCARSTYYGGDWYFNVWGAGTTVTVS AASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKAEPKSCDKTHTCPPCPAPELLG GPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGKHRRRKRSLDTSIIGGHEAKPHSRPYMAYL MIWDQKSLKRCGGFLIRDDFVLTAAHCWGSSINVTLGAHNIKEQEPTQQFIPVKRPIPHP AYNPKNFSNDIMLLQLERKAKRTRAVQPLRLPSNKAQVKPGQTCSVAGWGQTAPLGKHSH TLQEVKMTVQEDRKCESDLRHYYDSTIELCVGDPEIKKTSFKGDSGGPLVCNKVAQGIVS YGRNNGMPPRACTKVSSFVHWIKKTMKRY |
| 8 | GranzymeB | | IIGGHEAKPHSRPYMAYLMIWDQKSLKRCGGFLIRDDFVLTAAHCWGSSINVTLGAHNIK EQEPTQQFIPVKRPIPHPAYNPKNFSNDIMLLQLERKAKRTRAVQPLRLPSNKAQVKPGQ TCSVAGWGQTAPLGKHSHTLQEVKMTVQEDRKCESDLRHYYDSTIELCVGDPEIKKTSFK GDSGGPLVCNKVAQGIVSYGRNNGMPPRACTKVSSFVHWIKKTMKRY |
| 9 | Rituximab HCDR1 | | YTFTSYNMH |
| 10 | Rituximab HCDR2 | | WIGAIYPGNGDTSY |
| 11 | Rituximab HCDR3 | | RSTYYGGDWYFNV |
| 12 | Rituximab LCDR1 | | SSVSYIH |
| 13 | Rituximab LCDR2 | | PWIYATSNLAS |
| 14 | Rituximab LCDR3 | | QQWTSNPP |
| 15 | Glycosylation site | | EEQYNSTYR |
| 16 | Glycosylation site | | NFSNDIMLLQLER |
| 17 | Granzyme B - FCS-FC-VH-VL | 9G | IIGGHEAKPHSRPYMAYLMIWDQKSLKRCGGFLIRDDFVLTAAHCWGSSINVALGAHNIK EQEPTQQFIPVKRPIPHPAYNPKNFANDIMLLQLERKAKRTRAVQPLRLPSNKAQVKPGQ TCSVAGWGQTAPLGKHSHTLQEVKMTVQEDRKCESDLRHYYDSTIELCVGDPEIKKTSFK GDSGGPLVCNKVAQGIVSYGRNNGMPPRACTKVSSFVHWIKKTMKRYHRRKRSLDTSDK THTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSDG SFFLYSKLTVDKSRWQQGNVFSCSVMHEALHNHYTQKSLSLSPGGGGGSQIVLSQSPAILS ASPGEKVTMTCRASSSVSYIHWFQQKPGSSPKPWIYATSNLASGVPVRFSGSGSGTSYSL TISRVEAEDAATYYCQQWTSNPPTFGGGTKLEIKGGGGSGGGGSGGGGSQVQLQQPGAEL VKPGASVKMSCKASGYTFTSYNMHWVKQTPGRGLEWIGAIYPGNGDTSYNQKFKGKATLT ADKSSSTAYMQLSSLTSEDSAVYYCARSTYYGGDWYFNVWGAGTTVTVSA |
| 18 | Furin Cleavage site | | HRRRKRSLDTS |

## Claims

1. A method of producing a binder-toxin fusion protein comprising at least:
a) one protein binder selected from the group consisting of
• an antibody
• an antibody fragment or derivative retaining target binding capacity, and
• an antibody mimetic,
b) a non-cleavable peptide linker, and
c) at least one protein toxin,
said method comprising the steps of:
(i) contacting a plant cell or a whole plant with a nucleic acid construct comprising in operational linkage at least the following
A) at least one polynucleotide encoding for the protein binder, and
B) a polynucleotide encoding for the non-cleavable peptide linker and a polynucleotide encoding for a protein toxin,
(ii) allowing the construct to integrate into the nucleus of the plant cell, or of one or more cells of the whole plant, and
(iii) expressing the fusion protein encoded by the nucleic acid construct.

2. The method according to claim 1, which further comprises the step of (iv) recovering and/or purifying the fusion protein expressed in step (iii).

3. The method according to claim 1 or 2, wherein the plant or plant cell is from the genus *Nicotiana.*

4. The method according to any one of the aforementioned claims, wherein at least one protein toxin or protoxin is an enzyme.

5. The method according to any one of the aforementioned claims, wherein, the protein toxin is at least one selected from the group consisting of
(1) Cell death inducing proteins,
(2) Protein synthesis inhibitors,
(3) Membrane perturbating proteins, and
(4) Cell division inhibiting proteins.

6. The method according to any one of the aforementioned claims, wherein at least one protein toxin is a mammalian toxin, preferably selected from the group of Granzymes, more preferably Granzyme B, or a fragment thereof that retains the toxic activity of said protein toxin.

7. A binder-toxin fusion protein produced with a method according to any one of claims 1 - 6.

8. A binder-toxin fusion protein comprising at least:
a) one protein binder selected selected from the group consisting of
• an antibody
• an antibody fragment or derivative retaining target binding capacity, and
• an antibody mimetic,
b) a non-cleavable peptide linker, and
c) at least one protein toxin,
wherein the binder-toxin fusion protein is encoded by a nucleic acid construct comprising in operational linkage at least the following
A) at least one polynucleotide encoding for the protein binder, and
B) a polynucleotide encoding for the non-cleavable peptide linker and a polynucleotide encoding for a protein toxin.

9. The binder-toxin fusion protein according to claim 8, which protein comprises at least one plant-specific N-glycan.

10. The binder-toxin fusion protein according to any one of claims 8 - 9, wherein the protein binder binds to human CD20.

11. A pharmaceutical composition comprising at least the fusion protein according to any one of claims 8 - 10, and optionally one or more pharmaceutically acceptable excipients.

12. The pharmaceutical composition according to claim 11, which is administered with one or more further therapeutically active compounds.

13. The pharmaceutical composition of claim 11 or 12, for use in the treatment of a human or animal subject
• suffering from,
• being at risk of developing, and/or
• being diagnosed for,
developing a neoplastic disease, or for the prevention of such condition.
